# EUROPEAN PATENT APPLICATION

(11) **EP 4 275 581 A1**
(43) Date of publication of application: **15.11.2023**
(21) Application number: 22207526.9
(22) Date of filing: 15.11.2022
(51) Int. Cl.: A61B 1/00, A61B 1/04, G01N 21/64, G02B 21/16

(54) **METHOD, PROCESSOR, AND MEDICAL FLUORESCENCE OBSERVATION DEVICE FOR TOGGLING IMAGES**

(30) Priority: 13.05.2022 EP 22173318; 13.05.2022 EP 22173325; 13.05.2022 EP 22173291; 08.08.2022 EP 22189296
(71) Applicant: Leica Instruments (Singapore) Pte Ltd, Singapore 608924 (SG)
(72) Inventor: THEMELIS, George, Singapore 608924 (SG); PENTARAKIS, Kyriakos, Singapore 608924 (SG); SPINK, Roger, Singapore 608924 (SG)
(74) Representative: Grünecker Patent- und Rechtsanwälte PartG mbB

(57) **Abstract**

The invention relates to an image processor (170) and/or a computer-implemented image processing method for a medical fluorescence observation device (100), such as a fluorescence microscope or a fluorescence endoscope. A digital white-light color image (114) of an object (106) recorded in a first imaged spectrum (202) and a digital fluorescence-light color image (112) of the object (106) recorded in a second imaged spectrum (222) are retrieved. The second imaged spectrum (222) overlaps a fluorescence emission spectrum (226) of at least one fluorophore (116) and is different from the first imaged spectrum (202). Both the first and the second imaged spectrum (202, 222) overlap the visible spectrum (212). In order to facilitate the handling of the medical observation device (100) and the migration to new fluorescence imaging technologies for a user, it is provided to output at least one digital output color image (160, 160-1, 160-11, 160-III) of the set (1000) of digital output color images (160) containing: a digital output color image (160, 160-1) generated by the image processor (170) from only the digital fluorescence-light color image (112); a digital output color image (160, 160-11) generated by the image processor (170) from only the digital white-light color image (114); and a digital output color image (160, 160-III) generated by the image processor (170) from a combination of the digital fluorescence-light color image (114) and the digital white-light color image (112). In order to toggle between the various output images, a display selection signal (900) is received from a selector device (165); and the at least one digital output color image (160, 160-1, 160-11, 160-III) is selected from the group for output depending on the display selection signal (900).

## Description

The claimed subject matter relates to a computer-implemented image processing method and to an image processor for generating one or more digital output color images of an object for display, in a medical fluorescence observation device, such as a fluorescence microscope or a fluorescence endoscope. The claimed subject matter further relates to a method for operating such a medical fluorescence observation device and to a medical fluorescence observation device itself.

In existing medical fluorescence observation devices, fluorescence and anatomy images are recorded by a single camera. Such a setup may be used in fluorescence imaging of pPIX after administration of 5-ALA for revealing tumors. The selection of optical filters is such that the camera captures both the fluorescence in the red wavelengths and a fraction of the excitation light in the blue wavelengths. The excitation light reveals some of the tissue anatomy, while the fluorescence is indicative of tumors. The optical filters used today are standardized for pPIX imaging. The setup may also be used for other fluorophores such as ICG.

Although this kind of fluorescence imaging offers significant help especially in neurosurgical oncology, it still suffers from a severe drawback: the blue fluorescence excitation light which is used to visualize tissue anatomy offers only a poor visualization because its intensity is very low and the anatomy is only shown in monochromic shades of blue. In particular, under blue light, bleedings are very hard or even impossible to see. Thus, this setup is suboptimal for live surgical guidance.

Other medical fluorescence observation devices use a gray scale camera to record fluorescence and a color camera to record the white-light image. In such a set-up, the fluorescence image and the white-light image are digitally mixed, and fluorescence is marked with pseudocolor. Here, the disadvantage is that the pseudocolor does not accurately represent the colors of fluorescence as they would be perceived by the human eye.

In view of the above, there is a need to provide a device and a method with an improved representation of images to facilitate the handling of the medical observation method and to help users migrating from different visualization technologies.

This need is addressed by an image processor for a medical fluorescence observation device, such as a fluorescence microscope or a fluorescence endoscope, the image processor being configured to:
- retrieve a digital white-light color image of an object recorded in a first imaged spectrum;
- retrieve a digital fluorescence-light color image of the object recorded in a second imaged spectrum, the second imaged spectrum overlapping a fluorescence emission spectrum of at least one fluorophore and being different from the first imaged spectrum, the first and the second imaged spectrum both overlapping the visible spectrum; and
- output at least one digital output color image of a set of digital output color images containing:
   - a digital output color image generated by the image processor from only the digital fluorescence-light color image;
   - a digital output color image generated by the image processor from only the digital white-light color image; and
   - a digital output color image generated by the image processor from a combination of the digital fluorescence-light color image and the digital white-light color image;
wherein the image processor is further configured to
- receive a display selection signal from a selector device; and
- select the at least one digital output color image from the set for output depending on the display selection signal.

Further, this need is addressed by a computer-implemented image processing method for a fluorescence observation device, such as a fluorescence microscope or a fluorescence endoscope, the computer-implemented image processing method comprising the steps of:
- retrieving a digital white-light color image of an object recorded in a first imaged spectrum;
- retrieving a digital fluorescence-light color image of the object recorded in a second imaged spectrum, the second imaged spectrum overlapping a fluorescence emission spectrum of at least one fluorophore and being different from the first imaged spectrum, the first and the second imaged spectrum both overlapping the visible spectrum; and
- outputting at least one digital output color image of the set of digital output color images containing:
   - a digital output color image generated by the image processor from only the digital fluorescence-light color image;
   - a digital output color image generated by the image processor from only the digital white-light color image; and
   - a digital output color image generated by the image processor from a combination of the digital fluorescence-light color image and the digital white-light color image;
wherein the computer-implemented image processing method further comprises the steps of:
- receiving a display selection signal from a selector device; and
- selecting the at least one digital output color image from the set for output depending on the display selection signal.

The above image processor and computer-implemented image processing method facilitate the handling of the medical observation device as the device may be customized to display certain digital output color images in a coloring scheme that the user may be more familiar with or that correspond to different image recording setups. The user-dependent display selection signal controls which of the digital output color images of the set is displayed.

The above solutions may be further improved by the following features, which may be added and combined independently of one another, and each of which has its own advantageous technical effect. Each of the following features may be used both for improving one of the above methods and/or for improving one of the above devices, independent of whether the particular feature is only mentioned in relation to a method or in relation to a device.

For example, the image processor may be configured to output simultaneously at least two digital output color images of the output set. This facilitates comparison of different image modalities and/or different color schemes or color conversions and thus the detection of certain features of the object.

The at least two output color images may be displayed in a non-overlapping manner, e.g. side by side or in a grid. The image processor may also be configured to determine and/or alter a location of an output color image on a display depending on a user selection, e.g. depending on a signal from a pointer device such as a mouse, joystick, mouthpiece, foot pedal, track pad or digital pen. For example, this may allow a user to manually overlay two or more images to e.g. directly compare the spatial extent of image features in different images.

As the first and the second spectrum each represents only a part of the visible spectrum, the colors of the materials and tissue types recorded in the digital white-light color image and the digital fluorescence-light color image may not look natural and/or cannot be clearly distinguished from one another given the perceptive qualities of the human eye. For adjusting the colors, a color conversion function may be used. Different color conversion functions may be used to highlight different materials or tissue types or to affect the colors of a color space in specific ways. This again is explained in more detail below.

According to one advantageous aspect, the image processor may comprise a set of color conversion functions, each color conversion function being configured to map a color of an input pixel to a different color of an output pixel; and may be configured to compute the digital output color image generated from only the digital fluorescence-light color image by applying at least one color conversion function of the set of color conversion functions to the fluorescence-light color image; to compute the digital output color image generated from only the digital white-light color image by applying at least one color conversion function of the set of color conversion functions to the white-light color image; compute the digital output color image generated from the combination of the digital fluorescence-light color image and the digital white-light color image by applying at least one color conversion function of the set of color conversion functions to the white-light color image, to the fluorescence-light color image and/or to the combination of the digital fluorescence-light color image and the digital white-light color image. The selectability of such digital output color images facilitates the interpretation of the image data.

Although the digital white-light color image typically records the anatomy of the surgical scene by recording reflectance it may also be used to record fluorescence or both fluorescence and the reflectance of the object illuminated with a fluorescence excitation spectrum. The fluorescence-light color image may either record the fluorescence of one or more fluorophores, but may also be used to record a reflectance image of the anatomy, if fluorescence is not used. By employing a separate color camera for the digital fluorescence on one hand and the white-light image on the other, a much more color-accurate natural-color output image may be provided, independent of what is recorded in the digital fluorescence-light color image and the digital white-light color image. As the digital output color image in this configuration contains mainly fluorescence, e.g. either fluorescence excitation light as illumination or fluorescence emission, the expression "digital fluorescence output color image" is used. The digital fluorescence output color image is thus just a special case of a digital output color image that may be generated from the digital white-light color image and the digital fluorescence-light color image.

Thus, according to one embodiment, the digital white-light color image may be representative of a reflectance image of the object under illumination with a fluorescence excitation spectrum of at least one fluorophore, and/or the digital fluorescence-light color image may be representative of the fluorescence emission of the at least one fluorophore. Typically, fluorescence excitation spectrum is limited to wavelengths, were absorption of light is high, resulting in a tinted illumination. For example, illumination with the fluorescence excitation spectrum of 5-ALA/pPiX is blue to the human eye. This combination corresponds to the image that is recorded by a single color camera that captures both the fluorescence excitation and the fluorescence emission.

According to another embodiment, the digital white-light color image may be representative of a reflectance image of an object under white-light illumination, and/or the digital fluorescence-light color image may be representative of the fluorescence emission of the at least one fluorophore. As is explained in greater detail below, this combination allows to faithfully represent both a natural-looking reflectance image, e.g. in surgery of the anatomy, and a clearly visible fluorescence image of certain areas of interest.

According to yet another embodiment, the digital white-light color image and the digital fluorescence-light color image may be representative of a reflectance image of an object under white-light illumination. This combination, which is also discussed in greater detail below allows to more accurately analyze image content, to improve color representation and resolution.

In one embodiment, the image processor may be part of a medical fluorescence observation device. The medical fluorescence observation device may have a white-light color camera for recording one or more digital white-light color images and a fluorescence-light color camera for recording one or more digital fluorescence-light color image. The digital white-light color image may comprise a plurality of first pixels and each first pixel may comprise a first set of color space coordinates. Further, the first imaged spectrum may overlap a fluorescence emission spectrum of at least one fluorophore of the one or more fluorescing fluorophores. In the digital fluorescence-light color image, one or more fluorescing fluorophores may be represented in the second imaged spectrum. The digital fluorescence-light color image may comprise a plurality of second pixels and each second pixel may comprise a second set of color space coordinates. The digital fluorescence output color image may comprise a plurality of output pixels. The image processor may be configured to compute a color of an output pixel by applying a color conversion function to an input union of the first set of color space coordinates of a first pixel and the second set of color space coordinates of a second pixel.

The computer-implemented image processing method may comprise the steps of generating a digital fluorescence output color image from the digital white-light color image and the digital fluorescence-light color image and computing a color of an output pixel by applying a color conversion function to an input union of the first set of color space coordinates of a first pixel and the second set of color space coordinates of a second pixel.

The above image processor and computer-implemented image processing method allow to display the fluorescence of the fluorophores more clearly. Both the digital white-light color image and the digital fluorescence-light color image contain information on the fluorescence in different spectra, namely, the first and the second imaged spectra. The input union comprises more color bands and thus more spectral information than each of the digital white-light color image and the digital fluorescence-image individually. The additional spectral information is a result from the first and the second spectrum being different.

The input union of the first and second set corresponds to a multispectral image of the one or more fluorescing fluorophores. Using the increased spectral resolution by treating the digital white-light color image and the digital fluorescence-light image jointly as a multispectral image allows to apply individual color conversions for different tissue types and fluorescence speeds. It further allows to more reliably detect fluorescence and to distinguish between different types of fluorescence, such as between the fluorescence of different fluorophores including a differentiation between excited fluorescence of fluorophores that were added to the object and auto-fluorescence of materials inherent in the object.

The input union comprises more color bands and thus more spectral information than each of the digital white-light color image and the digital fluorescence-image individually. The additional spectral information is a result from the first and the second spectrum being different.

The input union of the first and second set corresponds to a multispectral image of the one or more fluorescing fluorophores. Using the increased spectral resolution by treating the digital white-light color image and the digital fluorescence-light image jointly as a multispectral image allows to apply individual color conversions for different tissue types and fluorescence speeds. It further allows to more reliably detect fluorescence and to distinguish between different types of fluorescence, such as between the fluorescence of different fluorophores including a differentiation between excited fluorescence of fluorophores that were added to the object and auto-fluorescence of materials inherent in the object.

The image processor may be configured to carry out any of the above processing steps. The terms "image processor" and "data processing device" may be used synonymously.

For example, one embodiment may be concerned with an image processor for a medical fluorescence observation device, such as a fluorescence microscope or a fluorescence endoscope, the processor being configured to retrieve a digital white-light color image of an object recorded in a first imaged spectrum, the digital white-light color image comprising a plurality of first pixels, each first pixel comprising a first set of color space coordinates in a first set of color bands; to retrieve a digital fluorescence-light color image of the object recorded in a second imaged spectrum, the digital fluorescence-light color image comprising a plurality of second pixels, each second pixel comprising a second set of color space coordinates in a second set of color bands; wherein the second imaged spectrum overlaps a fluorescence emission spectrum of at least one fluorophore, wherein the second imaged spectrum is different from the first imaged spectrum, and wherein the first and the second imaged spectrum both overlap the visible spectrum; to generate a digital output color image from the digital white-light color image and the digital fluorescence-light color image, the digital output color image comprising a plurality of output pixels; wherein the image processor is configured to compute a color of an output pixel by applying a color conversion function to an input union of the first set of color space coordinates of a first pixel and the second set of color space coordinates of a second pixel, wherein the application of the color conversion function is depending on the color space coordinates in the input union.

Another embodiment may be concerned with a computer-implemented image processing method for a fluorescence observation device, such as a fluorescence microscope or a fluorescence endoscope, the computer-implemented image processing method comprising the steps of retrieving a digital white-light color image of an object recorded in a first imaged spectrum, the digital white-light color image comprising a plurality of first pixels, each first pixel comprising a first set of color space coordinates in a first set of color bands; retrieving a digital fluorescence-light color image of the object recorded in a second imaged spectrum, the digital fluorescence-light color image comprising a plurality of second pixels, each second pixel comprising a second set of color space coordinates in a second set of color bands; wherein the second imaged spectrum overlaps a fluorescence emission spectrum of at least one fluorophore, wherein the second imaged spectrum is different from the first imaged spectrum, and wherein the first and the second imaged spectrum both overlap the visible spectrum; generating a digital output color image from the digital white-light color image and the digital fluorescence-light color image, the digital output color image comprising a plurality of output pixels; wherein a color of an output pixel is computed by applying a color conversion function to an input union of the first set of color space coordinates of a first pixel and the second set of color space coordinates of a second pixel, wherein the color conversion function is applied depending on the color space coordinates in the input union.

The at least one fluorescing fluorophore may be contained in the object that is imaged by the medical observation device and, in particular, the white-light color camera and the fluorescence-light color camera. The object may contain or consist of biological tissue.

At least one of the digital white-light color image and the digital fluorescence-light color image may contain or, equivalent represent, a reflectance image of the object illuminated by light consisting of or comprising a fluorescence excitation spectrum. Preferably, this reflectance image is contained in the digital white-light color image in addition to the fluorescence of the at least one fluorophore. Thus, the first imaged spectrum may overlap the fluorescence excitation spectrum.

The image processor and the computer-implemented method stated above allow to perform different color conversions for different colors in the object. By making the application of the color conversion function dependent on the input union of the two sets of color space coordinates a much more accurate color conversion scheme is possible. A color-dependent conversion allows e.g. to assign different color regimes to different fluorescence colors and reflectance colors under fluorescence-excitation illumination.

According to a further aspect, the computer-implemented image processing method may include the step of demosaicing at least one of the digital white-light color image and the digital fluorescence-light color image. By demosaicing, color artifacts may be avoided. Preferably, demosaicing is performed before any color conversion function is applied.

The computer-implemented image processing method may further include the step of normalizing at least one of the digital white-light color image and the digital fluorescence-light color image. Normalization facilitates the joint processing of the digital white-light color image and the digital fluorescence-light color image.

According to one aspect, the computer-implemented image processing method may include the step of registering the digital white-light color image and the digital fluorescence-light color image. By registering, image features that are present in both the digital white-light color image and the digital fluorescence-light color image are represented in each image by the same size and orientation. After registering, the digital white-light color image and the digital fluorescence-light color image match each other. Pixels at the same location in the two registered images are therefore corresponding pixels.

Specifically, the digital white-light color image and the digital fluorescence-light color image may already be registered with respect to one another when they are retrieved by the image processor. Alternatively, the image processor may be configured to register the digital white-light color image and the digital fluorescence-light color image with respect to one another.

The registration should be performed prior to the application of the color conversion function and preferably after demosaicing. In the registered digital white-light and fluorescence-light images, the first pixel comprising the first set of color space coordinates and the second pixel comprising the second set of color space coordinates are corresponding pixels, and/or the output pixel and the first and second pixels are corresponding pixels.

In one embodiment, the fluorescence medical observation device may be operated in various operational modes, in which different image modalities are assigned to the digital fluorescence-light color image and the digital white-light color image in addition or as an alternative to both images representing fluorescing fluorophores.

For example, the fluorescence-light color camera may, in one such operational mode, record a reflectance color image under the same illumination as the digital white-light color image, in particular a standard illuminant. Such a fluorescence-light color image may complement the reflectance image recorded by the white-light color camera. Thus, the fluorescence-light color camera is not restricted to recording only fluorescence images. However, the fluorescence-light camera may be restricted to only record images in the second imaged spectrum if the hardware is not changed, e.g. by exchanging one or more filters. In another operational mode, the digital white-light color image may represent a reflectance image of the object illuminated by a standard illuminant and the digital fluorescence-light color image may represent one or more fluorescing fluorophore. In all these operational modes, the generation of the output color image may be the same.

According to another advantageous embodiment of the computer-implemented image processing method and/or the image processor, the first imaged spectrum and the second imaged spectrum are complementary to each other, i.e. do not overlap. In such a configuration, crosstalk between fluorescence-light and white-light is avoided and an exact rendition of the fluorescence of the at least one fluorophore on one hand, and the reflectance of the object on the other, is maintained. Further, by having complementary spectra in the digital fluorescence-light color image and the digital white-light color image, respectively, the joint processing of the input union of the two images to form a multispectral image is facilitated, as there is no redundant special information.

The digital white-light color image may be represented in a first color space using at least three first color bands. The digital fluorescence-light color image may be represented in a second color space comprising at least three second color bands. The first and the second color spaces are preferably the same, such that the first and second color bands are also the same. However, this does not need to be the case. The first and the second color spaces can also be different.

The digital output color image may be generated in a third color space comprising at least three third color bands. The third color space may be the same color space as the first and/or the second color space, or be a different color space.

Any of the first, second and/or third color space may be an RGB color space, comprising three color bands R, G, B, or any other color space, such as HSV, LAB, CIELAB, XYZ, CMYK, multi-spectral color space or a hyperspectral color space, i.e. employ more than three color bands. The term "color" is used to designate the coordinates in the color space, i.e. the tuple of color space coordinates in the color bands of the color space, wherein a color band does not necessarily represent chroma but may also represent other aspects of the color, such as saturation, luminance or lightness as in a HSV, LUV or LAB color space. In this respect, the term "color band" is used synonymously for color space dimension throughout this text.

According to an aspect of the computer-implemented image processing method and/or the image processor, the first color bands and the second color bands are mapped onto the third color bands of the digital output color image using a color conversion function. The color conversion function may be non-linear, but preferably is a linear transformation. The color conversion function may comprise a color conversion matrix or other type of linear function. To map the first and second color bands to the third color bands, the color conversion matrix may be applied to the first color bands and the second color bands. Preferably, each color band of the first (set of) color bands and each color band of the second (set of) color bands is input as a separate color band into the linear transformation. In particular, each color band of the first color bands and each color band of the second color bands may be input simultaneously into the linear transformation.

The color conversion function may be stored in a memory of the image processor or the medical fluorescence observation device. As is explained further below, more than one color conversion function may be provided.

The color conversion matrix may have a dimension of a number X in one direction and dimension of a number Y in another direction. The number X may be the sum of the quantity of the first color bands, i.e. the number of color bands in the first set of color bands, and the quantity of the second color bands, i.e. the number of color bands in the second set of color bands. The number Y may be the quantity of color bands in the third color space. In other words, one dimension of the color conversion matrix may correspond to the sum of the dimensions of the color spaces of the digital white-light color image and the digital fluorescence-light image. Another dimension of the color conversion matrix may correspond to the dimension of the color space of the digital output color image. For example, if the first, second and third color spaces are each an RGB color space, the color conversion matrix may have a dimension 6x3 or 3x6 or 3x3.

The color conversion matrix may result from a combination of various matrices that are successively applied to the union set.

For example, a first, 6x1, intermediate matrix may be applied to the digital white-light color image and the digital fluorescence-light color image. This may be considered as reducing the color information in the union set to an intensity value. Such an intensity value may e.g. correspond to the fluorescence intensity of a particular fluorophore. A second, 1×3, intermediate matrix may then be used to map the intensity value to a color in the digital output color image. The application of these two matrices then corresponds to the application of a 6x3 matrix. Of course, the two intermediate

Thus, the third color bands, i.e. the color bands of the digital output color image, may result from a linear combination, in particular an addition, of the color bands of the digital white-light color image and the color bands of the digital fluorescence-light color image. Such a linear combination or addition can be considered a color conversion function as the two colors of the pixels in the digital white-light color image and the digital fluorescence-light color image are mapped onto the color of the pixel in the output image.

According to one aspect, the digital fluorescence-light color image and the digital white-light color image are processed jointly as a multispectral image of which the color bands are formed or constituted by the complementary color bands of the digital fluorescence-light color image and the digital white-light color image. This leads to improved color accuracy, especially if the fluorescence-light image represents reflected white light that has been recorded in the second imaged spectrum and thus may complement the white-light information in the white-light color image. Using this approach, the white-light information is contained in the color bands of the digital fluorescence-light color image and thus at a finer color granularity than just the digital white-light color image would offer. If the digital fluorescence-light color image represents fluorescence emission, treating the digital white-light color image and the digital fluorescence-light color image as a multispectral image represents a true representation of the object under both reflected white light and fluorescence. Especially if the digital fluorescence-light color image represents a reflectance image of the object under the same illumination as the digital white-light color image, additional spectral information is available.

According to another advantageous embodiment, the first imaged spectrum may comprise a first sub-band in a color band of a color space, the second imaged spectrum may comprise a second sub-band in this color band, wherein the first and the second sub-band are preferably complementary to one another. The color space may be the color space of the digital white-light color image, the color space of the digital fluorescence-light color image or, preferably, the color space of the digital output color image. It is preferred that the sub-bands of one color-band, together, complete the respective color band. This ensures that no useful spectral information is lost.

There may be a first and second sub-band in more than one color band of the respective color space. A sub-band in a color band may further be divided into a plurality of distinct wavelength bands that are separate from each other.

Preferably, the second imaged spectrum comprises an IR wavelength. This allows fluorescence emission in the near infrared to be captured or, if the digital fluorescence-light color image is not used for capturing fluorescence, may add additional information about the anatomy. Further, the IR wavelengths may contain spectral information that allows to better discern specific tissue types and the specific color-conversion functions assigned to them.

The image processor may be configured to generate the output pixel by applying the color conversion function simultaneously to both the first pixel and the second pixel.

According to an embodiment, the image processor may comprise at least two different color conversion functions. The image processor may be configured to select one of the at least two different color conversion functions depending on the input union, or in the method, one of the at least two different color conversion functions may be selected depending on the input union

Different color conversion functions may be used for different tissue types, where a tissue type comprises a predetermined range of colors. The predetermined range of colors may correspond one or more regions in the color space, where each region is characterized by a set of color space coordinates. The range of colors may be determined for each tissue type in a calibration process.

In order to assign different color conversion functions to different tissue types, the image processor may comprise at least two sets of different target unions. Each of the at least two different color conversion function may be assigned to a different set of the at least two sets of different target unions. The different target unions of a set correspond to the colors that are to be converted using the color conversion function assigned to the set. Each set of target unions may, for example, represent a different tissue type and/or the fluorescence emission of a different fluorophore and each target union in the set may correspond to a color that has been assigned to this tissue type or fluorescence during calibration.

To determine, in one embodiment, whether the input union is comprised in the set of predetermined target unions, the image processor may be configured to compare the input union to the target unions in a set of different target unions.

In one embodiment, the image processor may be configured to select a color conversion function from the set of at least two different color conversion functions depending on the union of the first set of color space coordinates of the first pixel and of the second set of color space coordinates of the second pixel is assigned.

The processor may be configured to select the color conversion that is assigned to the set of target unions which set contains the input union. This color function may then be applied to the input union.

In one embodiment, the image processor comprises at least one of:
- a predetermined set of target unions in which at least one target union corresponds to a color which is representative of oxygenated blood; such a set may represent tissue containing oxygenated blood, e.g. arterial tissue;
- a predetermined set of target unions in which at least one target union corresponds to a color which is representative of de-oxygenated blood; such a set may represent tissue containing de-oxygenated blood, e.g. venous tissue;
- a predetermined set of target unions in which at least one target union corresponds to a color which is representative of live grey brain matter;
- a predetermined set of target unions in which at least one target union corresponds to a color which is representative of live white brain matter;
- a predetermined set of target unions in which at least one target union corresponds to a color which is representative of the fluorescence of the fluorophore;
- a predetermined set of target unions in which at least one target union corresponds to a color which is representative of auto-fluorescence of biological tissue;
- a predetermined set of target unions in which at least one target union corresponds to a natural color which is representative of a color of biological tissue illuminated by a standard illuminant.

A target union maps the color represented in an input union set to a different output color. The target unions in a set may be determined e.g. by spectral decomposition, principal component analysis and/or color calibration.

For example, a set of target unions may be used to map a color in a reflectance image of the object under illumination of a fluorescence-excitation spectrum to a (more) natural color. The fluorescence-excitation spectrum contains or, preferably, consists of wavelengths that are shorter than the fluorescence emission wavelengths of the at least one fluorophore. Further, the fluorescence-excitation spectrum preferably has peaks at or in the region of wavelengths, where light absorption of the one or more fluorophores is maximum. This leads to a heavily tinted illumination of the object by the fluorescence-excitation spectrum. If, for example, 5-ALA/pPiX is used as a fluorophore, the fluorescence-excitation spectrum will have a strong blue tint and the reflectance image of the object under this illumination will be bluish. Using a color conversion function and in particular a set of target unions, the bluish tint may be mapped to more natural looking colors.

According to one embodiment, the image processor may be configured to be selectively operated in one of a first operational mode and a second operational mode, the first operational mode comprising an image-combining step and the second operational mode comprising a color-conversion step and the image-combining step; wherein the image processor is configured, in the image-combining step, to generate the digital output color image by combining the digital white-light color image and the digital fluorescence-light color image; and wherein the image processor is configured, in the color-conversion step, to apply at least one color conversion function from the group containing: a first color conversion function, which is applied to the digital white-light color image, and a second color conversion function, which is applied to the digital fluorescence-light color image.

The computer-implemented image processing method may be configured to be selectively executed in one of a first operational mode and a second operational mode; wherein, in the first operational mode, an image-combining step is executed and, in the second operational mode, a color-conversion step and the image-combining step are executed; wherein, in the image combining step, the digital output color image is generated by combining the digital white-light color image and the digital fluorescence-light color image and, in the color-conversion step, at least one color conversion function from the group containing: a first color conversion function, which is applied to the digital white-light color image and a second color conversion function, which is applied to the digital fluorescence-light color image, is applied.

By providing two operational modes that can be selected, it is both possible to offer a digital output color image in the fist operational mode that corresponds to what is seen through the ocular of the medical observation device.

By providing two color conversion functions which operate separately on the digital white-light color image and the digital fluorescence-light color image, respectively, it is possible to decouple the color conversion of the digital white-light image from the color conversion that is applied to the digital fluorescence-light color image. This allows to independently optimize each of these two images for viewing before they are combined to form the digital output color image. The first and/or second color conversion function is, according to one aspect, configured to shift a (recorded) color to a (different) color in the color space in the respective one of the digital white-light color image and the digital fluorescence-light image. The shift is predetermined by the color conversion function with respect to at least one of the amount and the direction of shift in the color space.

For example, the natural color of oxygenated or deoxygenated blood may only be incompletely captured in the first and second imaged spectrum respectively. This may particularly be the case if the reflectance spectrum of blood extends to wavelengths that are not recorded in the first or second imaged spectrum respectively. Therefore, if blood is recorded in the digital white-light color image or the digital fluorescence-light image, its color will not look natural, rendering it more difficult for a user or even an automatic image processing device to recognize a blood vessel. By using a first and/or a second color conversion function, the (recorded) color, here of blood, may be shifted to a more natural color in the respective digital white-light or fluorescence-light color image.

In another example, the recorded colors of oxygenated blood and deoxygenated blood may each be shifted differently by using different color conversion functions. Again, both theses colors are represented incompletely in the first and/or second imaged spectrum. The recorded color of de-oxygenated blood may be shifted further into the blue, whereas the color of oxygenated blood is shifted further into the red. This may require a different amount and/or direction of shift for the two recorded colors in the color space; one color may be shifted differently in the color space with respect to at least one of amount and direction than another color by the color conversion function.

The above example of course applies to any other type of tissue, such as fluid tissue like lymph, or solid tissue like bone, muscular and/or nerve tissue.

The converted color may correspond to the natural color, to a pseudocolor, such as a neon color, or to a "hyperreal" color, in which at least one of the color appearance parameters, namely, hue, colorfulness, saturation, lightness and brightness is modified compared to the natural color, in order to increase visibility and contrast.

The natural or true color in this context corresponds to the color that a CIE standard observer will perceive under a standard illuminant, such as a CIE illuminant A, B or C or any other standardized white-light illumination.

The first and/or second color conversion function may be configured to convert a color in the respective digital white-light or fluorescence-light color image to a color which is not located in the respective first and/or second imaged spectrum. Thus, the color conversion function may be used to correct deficits in the imaging which result from the limited first and/or second imaged spectrum. In another example, the first and/or second color conversion function may be configured to shift the white point of the digital white-light and/or fluorescence-light color image to a predetermined position, in particular to another white point. The white point, to which the white point is shifted to, may in particular correspond to a standardized white point as it is defied in a standard illuminant, such as a CIE illuminant. This allows to center the color space.

Further, the first and/or second color conversion function may be configured to white-balance or color-balance the respective the digital white-light and/or fluorescence-light color image. In a color balance, the intensity of the colors are adjusted to render at least some colors, in particular neutral colors, correctly. In a white balance, colors are adjusted to make a white object appear white and not colored.

According to another aspect, the first and/or second color conversion function may be configured to expand a contiguous or fragmented region in color space to a larger region in the respective digital white-light and/or fluorescence-light color image. Such a color conversion function allows to better visualize subtle differences between adjacent colors as adjacent colors are moved further apart. If, for example, the region of the fluorescence color of a particular fluorophore is expanded, nuances of the fluorescence become more visible. An expansion of the region may be accompanied of course by an appropriate color shift, so that the expansion of the region occurs about a natural or non-natural color.

According to yet another aspect, the first and/or second color conversion function may be configured to shift all colors in the color space of the respective digital white-light and/or fluorescence-light color image.

Any of the color conversion functions described above may be combined into a single color conversion function and/or any of the above color conversion functions may be applied sequentially to the respective one of the digital white-light and/or fluorescence-light color image.

It may be further advantageous, if the first and/or second color conversion function is configured to be applied dependent on the color of a respective pixel in the digital white-light color image and/or the digital fluorescence-light color image. This allows in general to treat different colors differently

According to another aspect, a plurality of different first and/or second color conversion functions may be provided and the processor may be configured to determine a first and/or second color conversion function of the plurality of different first and/or second color conversion functions which is applied to a pixel, dependent on the color of the pixel. The plurality of first and/or second color conversion functions may comprise any of the above-described color conversion functions. According to this aspect, targeted color-dependent modifications may be applied to the respective digital white-light and/or fluorescence-light color image. For example, the region around some colors in the digital white-light color image may be expanded in order to make color differences more visible. Another color, for example the colors of bone and/or nerves may simply be shifted to be more natural. Another color and thus the tissue associated with this color may be highlighted by being converted into a neon-color.

The above computer-implemented image processing method may be carried out when executing a method for operating a medical fluorescence observation device. The image processor as described above, may be part of a medical fluorescence observation device such as a fluorescence microscope or a fluorescence endoscope, in particular a fluorescence microscope or a fluorescence endoscope configured for surgery such as neurosurgery, orthopedic surgery and/or ophthalmic surgery. The fluorescence microscope may also be a laboratory microscope used, e.g. for biopsy.

A medical fluorescence observation device, such as a fluorescence microscope or a fluorescence endoscope, may comprise an image processor configured to carry out any of the above-described steps. Further, the medical fluorescence observation device may comprise a fluorescence-light color camera that is configured to record the digital fluorescence-light color image and a white-light color camera, which is configured to record the digital white-light color image.

As the fluorescence may be of lower intensity, the fluorescence-light color camera may be operated at a higher integration time.

The method for operating the medical fluorescence observation device may comprise the steps of recording the digital white-light color image using the white-light color camera and recording the digital fluorescence-light color image using the digital fluorescence-light color camera. The medical fluorescence observation device may comprise a digital fluorescence-light color camera and the white-light color camera. In particular, the fluorescence-light color camera and the white-light color camera may both record images of one or more fluorescing fluorophores. The white-light color camera may further record also a reflectance image of the object illuminated by the fluorescence excitation spectrum.

In order to reduce post processing, the fluorescence-light color camera and the white-light color camera may have overlapping coaxial and/or, preferably, identical field of views.

The medical fluorescence observation device may be a stereoscopic or monoscopic device. In one embodiment, a digital fluorescence-light color camera and a white-light color camera may be provided in each stereoscopic channel. Alternatively, in a monoscopic medical fluorescence observation device, only a single white-light color camera and a single digital fluorescence-light color camera may be provided.

According to one embodiment, the medical fluorescence observation device may comprise a selector device configured to be operated by a user of the medical fluorescence observation device and to generate the display selection signal.

The selector device may be a hardware device, such as a dial, knob or switch, a software deivce, such as an interactive graphical element which may resemble a dial, know or switch, or a combination of a software and a hardware device. The selector device may be used to generate the display selection signal. The selector device may be configured to generate a plurality of display selection signals, each of which may be uniquely assigned to a specific color conversion function or a specific combination of color conversion functions and a specific combination of the digital fluorescence-light color image and the digital white-light color image.

The medical fluorescence observation device may comprise at least one display, which is connected to the at least one image processor, the display being configured to receive and display the at least one digital output color image output by the image processor.

In an alternative arrangement of a stereoscopic medical fluorescence observation device, the fluorescence-light color camera may be provided in one stereoscopic channel, whereas the white-light color camera may be provided in the other stereoscopic channel. In such an arrangement, the fluorescence-light color camera may be configured to record (white-light) reflectance images in the second imaged spectrum. For (white-light) reflectance images, this arrangement provides a stereoscopic view, although strictly speaking, the stereoscopic view is limited to the overlapping parts of the first and second imaged spectrum, although this will be rarely noted by a human observer. In cases where fluorescence emission is recorded, the fluorescence-light color camera provides a monoscopic view on the fluorescence emission of the object, whereas the white-light color camera provides a monoscopic view on the reflectance of the object.

The medical fluorescence observation device may comprise an optical color separation assembly, which is configured to split the light entering color separation assembly into the first imaged spectrum and the second imaged spectrum. The color separation assembly may comprise optical elements such as a beam splitter, in particular a dichroic beam splitter, and/or optical filters such as a fluorescence-light filter blocking light outside the fluorescence emission and a white-light filter blocking the fluorescence emission.

The medical fluorescence observation device may further comprise an illumination assembly, which is preferably tunable, e.g. by comprising a plurality of differently-colored LEDs or OLEDs or other light sources which emit light in a plurality of different spectral bands and can be turned on and off individually.

To facilitate post-processing and to ensure that the recorded color space coordinates of the digital white-light image and the digital fluorescence-light color image are comparable to one another, it is preferred that the digital fluorescence-light color image and the digital white-light color image are recorded at the same time and/or use the same exposure time and/or use the same gain and/or the same white balance and/or color compensation. Moreover, the gain of the digital fluorescence-light color camera and the gain of the digital white-light color camera may be kept at a constant ratio, but otherwise allowed to adapt automatically. Of these possibilities, it is most preferred that the white-light color camera and the fluorescence-light color camera are synchronized with respect to at least one of exposure time and gain. However, the fluorescence-light color camera may be operated at a higher integration time. This allows to compensate for low fluorescence intensities. The white-light color camera and/or the fluorescence-light color camera is preferably a CCD or CMOS camera. The fluorescence-light camera and the white-light color camera are preferably identical.

According to another aspect, fluorophore-dependent sets of different color conversion functions may be provided, e.g. be stored in the image processor or medical fluorescence device. Each fluorophore-dependent set represents a different combination of a first and a second imaged spectrum, i.e. a different set of filters used in the color separation assembly, as is necessary if different fluorophores or combinations of different fluorophores are used. Examples of fluorophores used in this context are 5-ALA/pPIX, fluorescein and ICG. Each of these fluorophores requires excitation at different wavelengths and also emits fluorescence at different wavelengths. Thus, in a surgical environment in which ICG is used, the first imaged spectrum and the second imaged spectrum will be different to that of a surgical environment in which 5-ALA/pPIX is used, requiring a different color conversion matrix. Thus, the sub-bands of the first and second imaged spectrum in the color bands of the respective color spaces are different for each case of a fluorophore.

A fluorophore-dependent set may be automatically or manually selected from a plurality of such sets depending on the fluorophore or set-up of the color separation assembly used. For example, the medical fluorescence observation device may be configured to automatically detect the configuration or set-up of the color separation assembly and select a color conversion matrix depending on this set-up. If, for example, the optical filters of the color separation assembly for using ICG are replaced for using 5-ALA/pPIX, the medical fluorescence observation device may automatically select a color conversion matrix for this 5-ALA/pPIX.

The claimed subject matter also relates to a computer-readable medium and a computer program comprising instructions to cause a computer to carry out the computer-implemented image processing in any of the above embodiments.

As used herein, the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

In the following, the invention is described exemplarily with reference to embodiments and with reference to the drawings. The combination of features that are shown in the embodiments is not to be considered as limiting. For example, features of the embodiments which have a technical effect as e.g. explained above that is not needed in a specific application may be omitted. Conversely, features described above that are not part of an embodiment described below may be added if the technical effect associated with this particular feature is needed in a specific application.

Throughout the description and the drawings, the same reference numerals are used for elements that correspond to each other with respect to function and/or structure.

In the drawings,
- Fig. 1: shows a schematic representation of a medical fluorescence observation device;
- Fig. 2: shows a schematic representation of a combination of a digital white-light color image and a digital fluorescence-spectrum color image to arrive at a digital output color image;
- Fig. 3: shows a schematic representation of jointly processing a digital white-light color image and a fluorescence-light color image to arrive at a digital output color image;
- Fig. 4: shows a schematic representation of a tissue reflectance spectrum and spectral sensor sensivities;
- Fig. 5: shows schematic representations of the color conversion effected by various color conversion functions;
- Fig. 6: shows a schematic representation of a method for obtaining a digital output color image from a combination of a digital white-light color image and a digital fluorescence-spectrum color image; and
- Fig. 7: shows a schematic illustration of a system configured to perform a method for obtaining a digital output color image from a combination of a digital white-light color image and a digital fluorescence-spectrum color image;
- Fig. 8: shows a schematic illustration of applying one or more color conversion functions depending on an input union of the color bands of a pixel of a digital white-light color image and a pixel of a fluorescence-light color image;
- Fig. 9: shows a schematic illustration of different digital output color images obtained by applying different color conversion functions to the same digital white-light color image and the same digital fluorescence-light color image respectively;
- Fig. 10: shows a schematic illustration of a display displaying different combinations of the digital output color of Fig. 9.

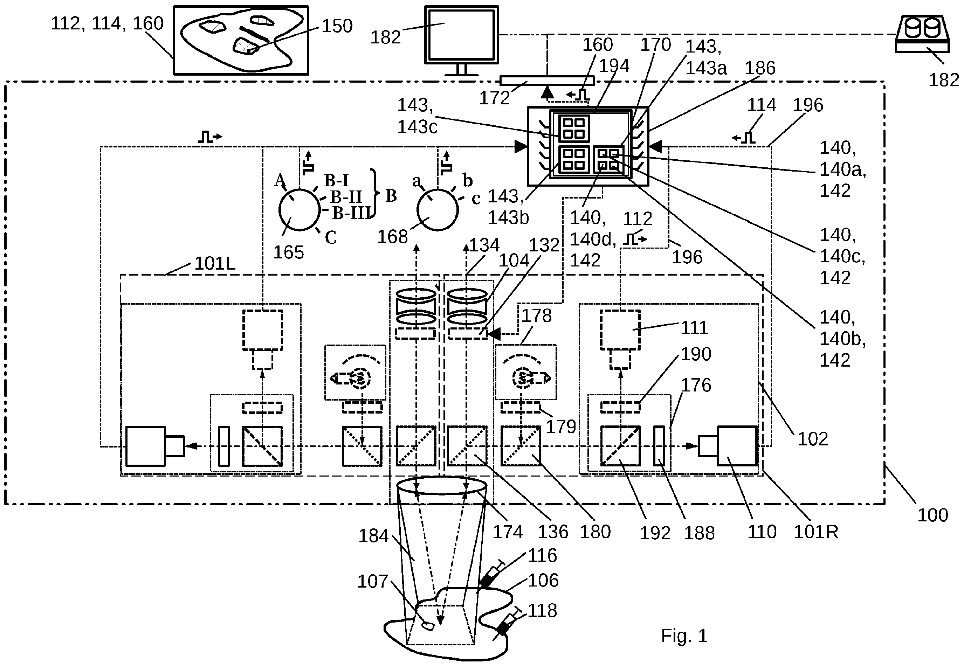

First, an exemplary embodiment of the invention is described with reference to Fig. 1.

Fig. 1 shows schematically a medical fluorescence observation device 100. The medical fluorescence observation device 100 may be a fluorescence microscope or a fluorescence endoscope, the difference between a microscope and an endoscope being primarily that, in an endoscope (not shown), an object 106 is viewed through optical fibers that are brought into vicinity of the object 106 to be investigated, e.g. by insertion into a body, whereas, in a microscope, an objective 174 is directed onto the object. Although the medical fluorescence observation device of Fig. 1 is a microscope, the following description also applies to an endoscope. The medical fluorescence observation device 100 may be a medical fluorescence observation device used in surgery. The medical fluorescence observation device 100 may also be a medical fluorescence observation device used in a laboratory, such as a laboratory microscope. The object 106 to be investigated may consist of or comprise biological tissue 107.

The object 106 may comprise one or more fluorophores 116, 118. The at least one fluorophore may be a fluorophore that is naturally contained in the object. For example, bone and blood contain fluorophores. The at least one fluorophore may also be added to the object 106, e.g. by injecting it into the biological tissue 107. Examples of fluorophores that may be added to the object 106 are ICG, fluorescein and/or 5-ALA. 5-ALA is synthesized in cells to pPIX/pPIX.

The medical observation device 100 is a fluorescence device. That means that the medical fluorescence observation device is configured to view, record and preferably also excite the fluorescence of the one or more fluorophores 116, 118.

The medical fluorescence observation device 100 may be a stereoscopic device as is exemplarily shown in Fig. 1. It may thus comprise two identical subassemblies 101L and 101R for each of the two stereoscopic channels. As the two subassemblies 101L, 101R are identical with respect to function and structure, the following description focuses on the right subassembly 101R, but applies identically to the left stereoscopic channel 101L.

The medical fluorescence observation device 100 may alternatively be a monoscopic device. In this case, only one of the two subassemblies 101L, 101R may be present. For a monoscopic medical fluorescence observation device 100, the following description therefore applies as well.

The medical fluorescence observation device 100 may be used to generate at least one digital white-light color image 114 which represents a reflectance image of the object 106 across the visible light range. The visible light range or visible spectrum comprises the wavelengths from about 310 nm to about 1100 nm, or from about 380 nm to 750 nm, or from about 450 nm to about 700 nm. The fluorescence spectrum or, if more than one fluorophore is used, the fluorescence spectra of the fluorophores 116, 118 is preferably omitted from the spectrum recorded in the digital white-light color image 114. This makes sure that only reflected light is contained in the digital white-light color image 114 if fluorescence is present. For example, if 5-ALA/pPIX is used as a fluorophore, the fluorescence spectrum from about 625 nm to about 650 nm may not be recorded in the digital white-light color image 114.

If light of specific wavelengths is used to excite fluorescence, the spectrum comprising or consisting of these wavelengths may also not be recorded or represented in the digital white-light color image 114. For example, if 5-ALA/pPIX is used as a fluorophore, fluorescence may be excited by illuminating the object 106 with wavelengths between about 380 nm to about 450 nm. For fluorescein and ICG and other fluorophores, different but known ranges for the excitation and emission spectra apply than for 5-ALA/pPIX.

By not recording the fluorescence-excitation spectrum and the fluorescence-emission spectrum, the digital white-light color image 114 preferably represents the reflectance of the object 106, i.e. represents a white-light image of the object 106 as it would be seen by a human observer. Thus, the digital white-light color image 114 may be regarded as a true-color image. However, in some embodiments, the emission spectrum or at least part of the emission spectrum of some fluorophores may also be recorded in the digital white-light color image 114.

The digital imaging system 102 may further be used to generate a digital fluorescence-light color image 112 of the object 106. The digital fluorescence-light color image 112 represents the fluorescence emission of the one or more fluorophores 116, 118. The digital fluorescence-light color image 112 therefore does preferably not record any wavelengths outside the emission spectrum or the emission spectra of the one or more fluorophores.

The digital white-light color image 114 and the digital fluorescence-light color image 112 are both color images. They are recorded using at least three color bands or, equivalently, primary colors of a color space. For example, the digital white-light color image 114 and the digital fluorescence color image 112 may be recorded in RGB color space using the three primary colors or color bands R, G, B. Alternatively, the digital white-light color image 114 and the digital fluorescence color image 112 may be recorded in different color spaces, respectively, and/or represent multi-spectral or hyperspectral color images. The digital white-light color image 114 and the digital fluorescence color image 112 need not be recorded in the same color space, although this is preferred.

The digital white-light color image 114 and the digital fluorescence-light image 112 contain pixels 150. In a color space such as an RGB color space, each color of a pixel 150 is represented by a triplet of three integer numbers, herein each integer number indicates the intensity of one of the primary colors R, G, B. For example, the most intense red may be indicated by the triple {255 ,0, 0}. The most intense green color may be indicated by {0, 255, 0}, and the most intense blue by {0, 0, 255}. Thus, RGB color space is a three-dimensional space, CMYK color space would be a four-dimensional space. A color can be considered as a point in color space having color space coordinates such as {0, 0, 255}. A multi-spectral or hyper-spectral color space having n color bands would correspondingly result in an n-dimensional color space, and each color would be represented by an n-tuple of values.

The spectrum recorded and represented in the digital white-light color image 114, the first imaged spectrum, and the spectrum recorded in the digital fluorescence-light color image 112, the second imaged spectrum, are preferably complementary to one another, i.e. do not overlap, except for unavoidable filter leakage. Preferably, they together represent the complete visible-light spectrum.

More specifically, the medical observation device 100 may comprise a digital imaging system 102 for generating the digital fluorescence-light color image 112 and the digital white-light color image 114. The digital imaging system 102 may comprise a white-light color camera 110 and a fluorescence-light color camera 111.

The white-light color camera 110 is configured to record the digital white-light color image 114. In particular, the white-light color camera 110 may be configured to generate a stream of digital white-light color images 114 in the form of a digital video stream. The white-light color camera 110 is preferably configured to record a digital image across the entire visible spectrum in the wavelengths indicated above. The white-light color camera 110 may be a CCD, CMOS or multi-spectral or hyperspectral camera.

The fluorescence-light color camera 111 is configured to record the digital fluorescence-light image 112. In particular, the fluorescence-light camera 111 may be configured to generate a stream of digital fluorescence-light color images 112 in the form of a digital video stream. The fluorescence-light color camera 111 may be configured to record the digital fluorescence-light color image 114 only in the fluorescence spectrum or the fluorescence spectra of the at least one fluorophore 116, 118. The fluorescence-light camera 111 may be configured to record the digital fluorescence-light image only in one or more narrow bands of light. These narrow bands should overlap the fluorescence spectrum or spectra of the one or more fluorophores 116, 118 of which fluorescence is to be recorded. Preferably, the fluorescence spectra of the fluorophore 116 and the second fluorophore 118 are at least partly separate, preferably completely, so that the fluorescence-light camera 111 may record light in two separate fluorescence bands that are spaced from one another.

The fluorescence-light color camera 111 may be a CCD, CMOS or multispectral or hyperspectral camera. Preferably, the white-light color camera 110 and the fluorescence-light color camera 111 are of the same type, although this is not necessary. As the fluorescence light has usually a very low intensity, the fluorescence-light color camera 111 may have a higher integration time.

The respective fields of view 184 of the cameras 110, 111 are preferably aligned or even coinciding and coaxial. Thus, it is preferred that the cameras 110, 111 provide the identical field of view 184 with the identical perspective and focal length. This results in identical representations of the object 106 in the images 112, 114 generated by the different cameras 110, 111. Both cameras 110, 111 may use the same objective 174.

If a match of the perspectives and field of view cannot be generated optically, it may be generated by image processing by applying a matching or registering routine to the digital images 112, 114, as is explained further below. Registering may also take place if the cameras 110, 111 have identical perspectives and fields of view.

It is preferred that the two cameras 110, 111 are operated synchronously. Specifically, the exposure times may be synchronized. Thus, the medical fluorescence observation device 100 may be configured to generate the digital white-light color image 114 and the digital fluorescence-light image 112 at the same time.

Preferably, the gain of the two cameras 110, 111 is synchronized, i.e. adjusted in the two cameras 110, 111 at the same time. Moreover, the ratio of the gain applied in camera 110 to the gain applied in camera 111 may be constant, even if the gain is changed. The gamma correction and color adjustment or white balance may be switched off or kept constant.

Any of the above measures facilitates the comparison, joint processing and/or combining of the two images 112, 114

For separating the spectrum recorded in the digital white-light color image 114 from the spectrum recorded in the digital fluorescence-light color image 112, i.e. for separating the reflectance spectrum from the fluorescence spectrum, the medical observation device 100 may comprise an optical color-separation assembly 176. The color-separation assembly 176 may comprise optical elements such as a beam splitter 192, which may be dichroic. The color separation assembly 176 may further or alternatively comprise an optical white-light filter 188 and/or an optical fluorescence-light filter 190.

The fluorescence-light filter 190 is preferably configured to transmit light in the fluorescence spectrum or spectra of the one or more fluorophores 116, 118 and to block light outside the fluorescence spectrum or spectra.

The fluorescence-light filter 190 may be configured as a band-pass filter comprising one or more passbands. Each passband should overlap the fluorescence emission spectrum of a respective fluorophore 116, 118 of which the fluorescence is to be recorded. As the fluorescence-light filter 190 is in the light path between the beam splitter 192 and the fluorescence-light camera 111, only the wavelengths in the passbands of the fluorescence-light filter 190 are transmitted to the fluorescence-light camera 111.

The white-light filter 188 is preferably configured to block light in the fluorescence spectrum or spectra of the one or more fluorophores 116, 118. The white-light filter 188 may also be configured to block light in the fluorescence-excitation spectrum.

The white-light filter 188 is preferably configured as a band-stop filter, of which the stop bands correspond to or at least contain the passbands of the fluorescence-light filter 190. The white-light filter 188 is located in the light path between the beam splitter 192 and the white-light camera 110. Thus, white-light camera 110 records only wavelengths that are outside the stop-bands of the white-light filter 188 and therefore also outside of the pass-bands of the fluorescence-light filter 190 of the fluorescence-light filter 190.

Any one of the white-light filter 188 and the fluorescence-light filter 190 may be a tunable filter.

If the beam splitter 192 is a dichroic beam splitter, at least one of the filters 188, 190 may be omitted as the optical spectral filtering in this case is already integrated in the dichroic beam splitter. The above description of the passbands and stopbands then should apply mutatis mutandis to the dichroic beam splitter 192.

Thus, the white-light color camera 110 records the digital white-light color image 114 in a first imaged spectrum, the reflectance spectrum, which is different from a second imaged spectrum, which is recorded as fluorescence spectrum by the fluorescence-light camera. The wavelengths that are contained in the first and the second imaged spectrum are determined by the filter settings of the color separation assembly 176.

The medical fluorescence observation device 100 may further comprise an illumination assembly 178, which is configured to illuminate the object 106 preferably through the objective 174 through which the imaging system 102 records the at least one digital image 112, 114. The illumination assembly 178 may be configured to selectively generate white-light, i.e. light that is evenly distributed across the entire visible spectrum, and fluorescence-excitation light, which contains light only in wavelengths that stimulate fluorescence of the at least one fluorophore 116, 118. The illumination light generated by the illumination assembly 178 may be fed into the objective 174 using an illumination beam splitter 180.

An illumination filter 179 may be provided depending on the fluorophore and its fluorescence-specific excitation spectrum. For example, if 5-ALA/pPIX is used as a fluorophore, the illumination filter may have a transmission of 90 % to 98 % up to wavelengths of 425 nm, a transmission between 0.5 % and 0.7 % in wavelengths between 450 nm and 460 nm, a transmission of not more than 0.1 % between 460 nm and 535 nm and of practically zero for wavelengths above 535 nm.

Instead of or in addition to the illumination filter 179, the illumination assembly 178 may comprise a tunable light source, comprising e.g. a multitude of differently colored LEDs or OLEDs.

The medical fluorescence observation device 100 may further comprise an image processor 170. The image processor 170 may be a hardware module, such as a microprocessor, or a software module. The image processor 170 may also be a combination of both a hardware module and a software module, for example by using software modules that are configured to be run on a specific processor, such as a vector processor, a floating point graphics processor, a parallel processor and/or on multiple processors. The image processor 170 may be part of a general-purpose computer 186, such as a PC.

The image processor 170 is configured to retrieve the digital white-light color image 114 and the digital fluorescence-light image 112. For example, the image processor 170 may be configured to retrieve the digital white-light color image 114 and the digital fluorescence-light image 112 from a memory 194 and/or directly from the cameras 110, 111. The memory 194 may be part of the image processor 170 or reside elsewhere in the medical fluorescence observation device 100.

The image processor 170 is further configured to compute a digital output color image 160 from the digital white-light color image 114 and the digital fluorescence-light image 112. The digital output color image 160 is a color image which is represented in a color space. The color space of the digital output color image may be different from the color space of any of the digital white-light color image 114 and the digital fluorescence-light image 112. Preferably, however, the color space of the digital output color image 160 is the same color space as that of the digital white-light color image 114 and the digital fluorescence-light image 112.

The image processor 170 comprises at least two operational modes in which it is configured to operate selectively. For example, a user (not shown) may manually select which of the two operational modes is carried out. For selecting the operational mode from the at least two available operational modes, a selector device 165 may be provided. The selector device 165 may be a mechanical device, such as a dial or a switch, or a button or other element on a graphical user interface displayed on a display such as a display 132, 182, described in greater detail below.

As shown in Fig. 1, the image processor 170 may comprise a first operational mode A and a second operational mode B and, optionally, a third operational mode C. Alternatively, the image processor 170 may be configured to operate in only a single operational mode, e.g. in one of the modes A, B and C.

In the first operational mode A, the image processor 170 is preferably configured to generate a digital output color image 160 which represents what would be seen through the eyepiece 104. To achieve this, in operational mode A, the image processor 170 may be configured to combine the digital fluorescence-light color image 112 and the digital white-light color image 114 to generate the digital output color image 160. In particular, the image processor 170 may be configured to additively combine or to add, at each corresponding pixel 150 in the digital fluorescence-light color image 112 and the digital white-light color image 114, the values of the digital fluorescence-light color image 112 and the digital white-light color image 114 in each color band of their color space. From this combination, a color in an output pixel in the digital output color image 160 is generated

In the second operational mode B, the image processor 170 is configured to first apply a color conversion function 140 to at least one of the digital white-light color image 114 and the digital fluorescence-light color image 112 and then to generate the digital output color image 160 from the digital white-light color image 114 and the digital fluorescence-light color image 112. Thus, in the second operational mode B, at least one of the digital white-light color image 114 and the digital fluorescence-light color image 112 is color-converted before being combined. By switching between the first operational mode A and the second operational mode B, the user is able to switch between a view which faithfully renders what is seen through the eyepiece to a view which may highlight different features of the object 106.

The color conversion function 140 is configured to map a color of an input pixel, or of more than one input pixel, to a different color in an output pixel. Thus, the color conversion function, upon application, alters the input color(s) in a foreseeable manner. The color conversion function 140 may be any type of function such as a one- or n-dimensional interpolation function. Preferably, however, the color conversion function 140 is a linear transformation function. Specifically, the color conversion function 140 may be a color conversion matrix 142. One dimension of the color conversion matrix 142 may be the sum of the number of color bands in the digital fluorescence-light color image 112 and of the number of color bands in the digital white-light color image 114. Another dimension of the color conversion matrix 142 may correspond to the number of color bands in the digital output color image 160 in the other direction in the other direction of the matrix.

In another embodiment, the color conversion matrix 142 may have a first dimension which corresponds to the number of color bands in the digital white-light color image 114 or the digital fluorescence-light color image 112, depending on which one of those two the color conversion matrix is applied, and a second dimension which corresponds to the number of color bands in the digital output color image 160.

If all the images 112, 114 are in RGB color space, the dimension of the color conversion matrix 142 is preferably a 3x3 matrix, or a 6x3 or 3x6 matrix if the color conversion matrix is configured to operate simultaneously on the digital white-light color image and the digital fluorescence-light color image. The color conversion matrix may also result from the subsequent application of two or more matrices. For example, the color conversion matrix 142 may result from first applying a 6x1 color conversion matrix and then a 1x3 color conversion matrix, which has the same effect as applying a 6x3 color conversion matrix.

The second operational mode B itself may comprise different submodes. Each submode may apply a different color conversion function 140 to at least one of the digital white-light color image 114 and the digital fluorescence-light color image 112 before the digital fluorescence-light color image 112 is combined with the digital white-light color image 114. In Fig. 1 just by way of example, three submodes B-I, B-II, B-III are shown. More or less than three submodes may of course be provided. The different color conversions functions 140 may be stored in the memory 194.

For example a first color conversion function 140a may be applied to the digital white-light color image 114 only and a second color conversion function 140b may be applied only to the digital fluorescence-light color image 112 only. The first and the second color conversion function 140a, 140b, may be applied independently of another, decoupling the color conversion of the digital white-light color image 114 from the color conversion of the digital fluorescence-light color image 112. For example, only one of the first and the second color conversion function 140a, 140b may be applied to the respective image 112, 114 or both may be applied to their respective images.

The user may, in one embodiment, be able to select between different first color conversion functions 140a and/or different second color conversion functions 140b.

For example, in operational mode B-I a first color conversion function 140a may be used which is configured to convert a color in the digital white-light color image 112 to a color which is not located in the second imaged spectrum. In this case, a natural color of the object 106, or part thereof, which is not recorded correctly in the digital white-light color image 114, because it was partly filtered and therefore distorted by the color separation assembly 176, can be converted by the first color conversion function 140a to a color which more closely corresponds to the natural color.

In another additional or alternative example, a first color conversion function 140a may be used in operational mode B-I, or an additional operational mode, which converts a color in the digital white-light color image 114 to a pseudocolor or a different hue. This allows to highlight colors which are associated with certain type of tissues. For example nerves, the color of arterial blood and/or the color of venous blood all may be converted to different pseudocolors.

By using the selector device 165, the user may quickly go through different assignment of pseudocolors to different types of tissues. In one operational mode, the color of arterial blood may be converted to neon-red, in another operational mode, the color of venous blood may be converted to neon-blue, and in yet another operational mode, the color of nerve tissue may be converted to neon-yellow. Other operational modes may use combinations of these modes. If instead of a pseudocolor, the first color conversion function 140a assigns a different hue, contrasts may be enhanced. For example, the first color conversion function 140a may spread the red colors in the digital white-light color image 114 over a wider range, thus making smaller changes in the oxygenation of blood more visible to the user.

In yet another additional or alternative example, the first color conversion function may be used in e.g. operational mode B-I or an additional operational mode to color-balance and/or to white-balance the digital white-light color image 114. In a white-balance the overall mixture of colors is changed but the neutral colors, i.e. grey, black and white, are maintained as neutral colors. In a color-balance, the colors including the neutral colors are adjusted to correspond to the neutral colors.

In yet another additional or alternative example, the first color conversion function 140a may be used in e.g. operational mode B-I or an additional operational mode to shift the white point of the digital white-light color image 114 to a predetermined position in color space. Adjusting the white point allows to adjust for different illuminations and for the filter settings of the color separating assembly 176.

According to one example, at least one of the above described first color conversion functions 140a may be applied to the digital white-light color image 114. According to another example at least two of the above described first color conversion functions 140a may be applied sequentially to the digital white-light color image 114. Alternatively or additionally, two or more of the above described color conversion functions 140a may be combined into a single color conversion function 140a.

The descriptions above of the various first color conversion functions 140a also apply mutatis mutandis to the various second color conversion functions 140b, the only difference between these two functions being that the second color conversion 140b is configured to operate on the digital fluorescence-light image 114 and to operate on different colors.

In an operational mode B-II, for example, one or more color conversion functions 140b may be applied to the digital fluorescence-light image only and no color conversion function 140a is applied.

In an operational mode B-III, for example, one or more color conversion functions 140a may be applied to the digital white-light color image and one or more color conversion functions 140b may be applied to the digital fluorescence-light color image 112.

An optional third operational mode C may be provided in which a third color conversion function 140c is applied simultaneously to the digital white-light color image 114 and the digital fluorescence-light color image 112, wherein application of the color conversion function 140c results directly in the digital output color image 160.

If another type of fluorophore is used, which has different excitation and/or fluorescence wavelengths, different color conversion functions 140a, 140b, 140c need to be provided, because the combination of filter settings in the color separation assembly 176 and thus the first and the second imaged spectrum need to be adapted to the excitation and/or fluorescence wavelengths.

Thus, in one embodiment, the medical observation device 100 or the processor 170 may be configured to store a plurality of different sets 143 of color conversion functions 140a, 140b and/or 140c. Each different set 143 comprises one or more of the above described color conversion functions 140a, 140b and /or 140c. Each different set 143 corresponds to the use of a different fluorophore and thus represents the different filter settings of the color separation assembly 176. For example, a first set 143a may be used for ICG as fluorophore, whereas a second set 143b may be used for 5-ALA/pPiX as fluorophore and of course further sets may be used to accommodate further fluorophores or combinations of fluorophores.

The medical fluorescence observation device 100 may be adjusted to a different fluorophore by re-configuring the color-separation assembly 176, e.g. by exchanging its optical elements, such as the filters 190 and/or 192, or the dichroic beam splitter 180. The selection of the appropriate set 143 of color conversion functions 140 may be performed automatically, e.g. if the medical observation device 100 is configured to automatically detect the set-up of the color separation assembly 176.

Alternatively or additionally, the medical observation device 100 may comprise a filter setting selector device 168 which allows a user to manually select the set 143 of color conversion functions 140 which is to be applied to the digital white-light color image 114 and/or the digital fluorescence-light color image. The filter setting selector device 168 may be mechanical and/or part of a graphical user interface. If for example, the filter setting selector device 169 is operated to be in a position a, the set 143a is selected. In a position b, the set 143b is selected and so on.

In another embodiment, the filter setting selector device 168 may allow a user to select different image constituents of the digital white-light color image 114 and the digital fluorescence-light color image 112. For example, in one position of the filter setting selector device 168, only the excited fluorescence of the one or more fluorophores is shown. In another position, only autofluorescence may be shown. And in again another position of the filter setting selector device, a combination of a reflectance image of the object illuminated with only the fluorescence excitation spectrum and the excited fluorescence of the at least one fluorophore 116, 188 may be shown.

The digital output color image 160 may be displayed on the display 132 which is integral with the medical fluorescence observation device 100. For example, the display 132 may be integrated in an ocular or eyepiece 104 of the medical fluorescence observation device 100. The display 132 may also display a graphical user interface for operating the medical observation device 100.

The medical fluorescence observation device 100 may comprise a direct optical path 134 from the object 106 through the objective 174 to the eyepiece 104. In such a case, the display may be a translucent display 132 located in the direct optical path 134 or the display may be projected into the direct optical path 134. A beam splitter 136 may be provided to split the light between the optical eyepiece 104 and the digital imaging system 102. In one embodiment, up to 80 % of the light may be directed to the eyepiece 104.

Alternatively, the medical fluorescence observation device 100 may not have a direct optical path 134 but only display images from the integral display 132. As a further alternative, the medical fluorescence observation device may not have any display at all.

The medical fluorescence observation device 100 may comprise an output interface 172 to which one or more (external) displays 182 may be connected. For this, the output interface 172 may comprise standardized connectors and data transmission protocols, such as USB, HDMI, DVI, DisplayPort, Bluetooth and/or others. An external display may be a monitor, 3D goggles, oculars and the like. Any combination of external displays may be connected to output interface 172. Any of the displays 182 may display a graphical user interface for operating the medical observation device 100.

The computer 186 or the image processor 170 is connected to the digital imaging system 102 using one or more data transmission lines 196. A data transmission line may be wired or wireless, or partly wired and partly wireless. The computer 186 and/or the image processor 170 may not be bodily integrated in the medical fluorescence observation device 100 but be physically located remote from the digital imaging system 102. For this, the digital imaging system 102 and the computer 186 and/or the image processor 170 may be connected to a network, such as a LAN, a WLAN or a WAN, to which also at least one display 182 is connected.

According to a modification, the medical fluorescence observation device 100 may be stereoscopic but comprise only two cameras, one for each stereoscopic channel. In one stereoscopic channel, the fluorescence-light color camera 111 is used and configured to selectively also record white-light reflectance, whereas in the other stereoscopic channel, the white-light color camera 110 is used. Such an arrangement provides a stereoscopic white-light color image if no fluorescence is used and a monoscopic white-light color image and a monoscopic fluorescence-light color image if fluorescence is used. The description above and below applies equally to this configuration.

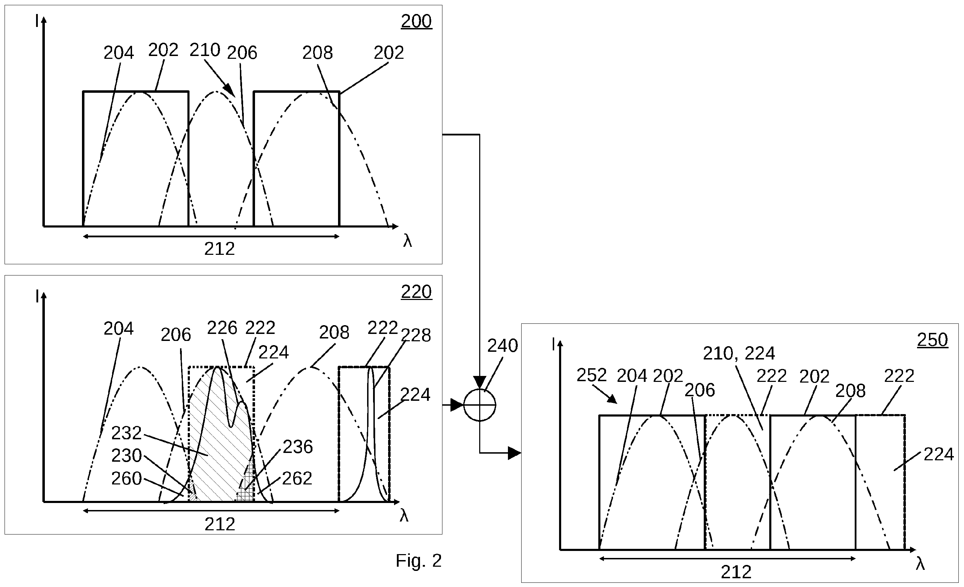

Fig. 2 shows an example of the first operational mode A.

Reference numeral 200 is a quantitative example of the first imaged spectrum 202 as it is recorded by the digital white-light camera 110 and/or represented in the digital white-light color image 114, respectively. The intensity I across the wavelengths/colors λ is shown as normalized. The first imaged spectrum 202 preferably extends at least across the visible spectrum 212.

Just by way of example, the color space in which the first imaged spectrum 202 is recorded, is an RGB color space having three primary colors or color bands 204, 206 and 208. One primary color 204 is blue, another primary color 206 is green and a third primary color 208 is red. The sensitivities of the sensors of the white-light color camera 110 in the different primary colors 204, 206, 208 are tuned to result in a sensitivity across the visible spectrum 212 which is as constant as possible.

If a color space other than RGB is used, the number, location and/or width of the color bands may be different. Other than that there is no principal difference to RGB color space.

The first imaged spectrum 202 does not include the fluorescence-excitation light and the fluorescence emission spectrum of the at least one fluorophore 116, 118. Thus, the first imaged spectrum 202 may include at least one stop band 210 which coincides with the fluorescence emission of the at least one fluorophore of which fluorescence is to be recorded by the fluorescence-light color camera 111. The stop band 210 is e.g. created by the white-light filter 188. The number, width, and/or location of stop bands 210 depend on the number and types of fluorophores to be observed in the object 106.

At reference numeral 220, the second imaged spectrum 222 is shown as it is recorded by the fluorescence-light color camera 111 and/or represented in the digital fluorescence-light color image 112, respectively. Just by way of example, the color space in which the second imaged spectrum 222 is recorded is also an RGB color space. The sensitivities of the sensors of the fluorescence-light color camera 111 in the different primary colors 204, 206, 208 are tuned to result in a sensitivity across the visible spectrum 212 which is as constant as possible.

The spectra 202, 222 do not need to be recorded in the same color space, although this is preferred.

The second imaged spectrum 222 may comprise one or more passbands 224. The number, location and/or width of the passbands depends on the number and types of fluorophores used. The at least one passband 224 preferably corresponds to the at least one stop band 210. The at least one passband is e.g. generated by the fluorescence-light filter 190.

The first imaged spectrum 202 and the second imaged spectrum 222 are complementary to one another. They preferably complete each other to cover the entire or most of the visible spectrum 212.

Each passband 224 of the second imaged spectrum 222 preferably overlaps the fluorescence-emission spectrum 226, 228 of a fluorophore 116, 118, of which fluorescence is to be recorded, and overlap one or more primary colors 204, 206, 208 of the color spectrum. For example, the fluorescence-emission spectrum 226 of one fluorophore 116 may overlap all three primary colors 204, 206, 208. The sensor of the fluorescence-light camera 111 recording the color band 204 only records a small part 230 of the fluorescence-emission spectrum 226. The sensor of the fluorescence-light camera 111 recording the color band 206 records a majority 232 of the fluorescence-emission spectrum 226, whereas the sensor of the fluorescence-light camera 111 recording the color band 208 again only records a small part 236 of the fluorescence-emission spectrum 226.

The fluorescence spectrum 228, if present in a particular case, in contrast is only recorded by the fluorescence-light camera 111 recording the color band 208.

Some fluorophores, such as 5-ALA/pPiX may have a very wide fluorescence emission spectrum 226. In such a case, it may not be advisable to extend the pass band 224 to cover the entire fluorescence emission spectrum as, otherwise, too many wavelengths will be missing in the white-light reflectance image. Therefore, the pass band 224 may not cover the entire fluorescence emission spectrum 226. Consequently, some fluorescence may be contained in the first imaged spectrum 202 and be recorded in the digital white-light color image 114. This does not impair the digital white-light color image 114 if recorded under a standard illuminant as a white-light reflectance image as the intensity of the fluorescence will be much weaker than the intensity of the reflected white light. Further, any fluorescence recorded in the digital white-light color image may be used to complement the fluorescence information in the digital fluorescence-light color image.

In Fig. 2, those parts of the fluorescence emission spectrum 226 that are contained in the first imaged spectrum 202 are shown at reference numerals 260 and 262 respectively. Reference numeral 260 designates wavelengths of the fluorescence emission spectrum 226 that are smaller than the lower cut-off wavelength of the passband 224. Reference numeral 260 designates wavelengths of the fluorescence emission spectrum 226 that are smaller than the higher cut-off wavelength of the passband 224.

The fluorescence emission in the first imaged spectrum 260 may be captured or recorded in all color bands 204, 206, 208. The first part 260 of the fluorescence emission spectrum 226 in the first imaged spectrum may e.g. overlap the color bands 204 and 206. The second part 262 may overlap color bands 206 and 208.

If the digital white-light color image 112 is not used for recording a white-light reflectance image but used for recording fluorescence only, e.g. if the object is not illuminated with a white-light standard illuminant but with a fluorescence excitation spectrum, containing only wavelengths that are shorter than the wavelengths in fluorescence emission spectrum, the digital white-light color image may also be representative of a fluorescence image of the object 106. In such a case, fluorescence information is contained in non-overlapping spectral bands in both the digital white-light color image 114 and the digital fluorescence-light color image 112.

The fluorescence excitation light which illuminates the object may be blocked in the color separation assembly 176 e.g. by an appropriate filter 188 and/or 190, and/or an appropriate dichroic beam splitter 176. In another embodiment, however, the fluorescence excitation light may be recorded in at least one of the digital white-light color image 114 and the digital fluorescence-light color image 112. In the latter case, some reflectance information is preserved which may be used in post-processing.

In its simplest form, a combination 240 of the digital white-light color image 114 and the digital fluorescence-light color image 112 is an additive combination. For example, the values in each color band of the digital white-light color image 114 and the fluorescence color image 112 are added at each corresponding pixel 150. A corresponding pixel 150 may be a pixel 150 which is located at the same position in both images 112, 114, if the images 112, 114 are registered and of the same size. If the images 112, 114 are both RGB images and the values in the RGB color bands of a pixel 150 in the digital white-light color image are {R1, G1, B1}, and the values in the RGB color bands of the corresponding pixel 150 in the digital fluorescence-light color image are {R2, G2, B2}, then the values in the RGB color bands of the corresponding pixel 150 in the digital output color image are {R1+R2, G1+G2, B1+B2}. The combination 240 results in a color conversion, because, the color of the pixel of the output image is different from the colors of the pixels in the images 112, 114. The combination 240 therefore is an example of a color conversion function 140. The combination 240 may be implemented using a color conversion matrix 142.

Reference numeral 250 points to a qualitative rendition of the spectrum 252 of the digital output image 160. In essence, the spectrum 252 of the digital output image 160 is an addition of the first imaged spectrum 202 and the second imaged spectrum 222.

Alternatively, the individual spectra 202 and 222 are treated as a single, combined multispectral spectrum which has a number of color bands that corresponds to the sum of color bands in the first imaged spectrum 202 and the second imaged spectrum 222. In other words, the digital fluorescence-light color image 112 and the digital white-light color image 114 are jointly processed as a single (virtual) multispectral image. Instead of adding the color space coordinates in each color band of the images 112, 114, a union set is formed and processed, keeping the color bands of the images 112, 114 separate.

This corresponds to operational mode C (Fig. 1), where a color conversion function 140, 140c is applied to both the digital white-light color image 114 and the digital fluorescence-light color image 112 to obtain the digital output color image 160 of which the spectrum is indicated at reference numeral 250 in Fig. 2.

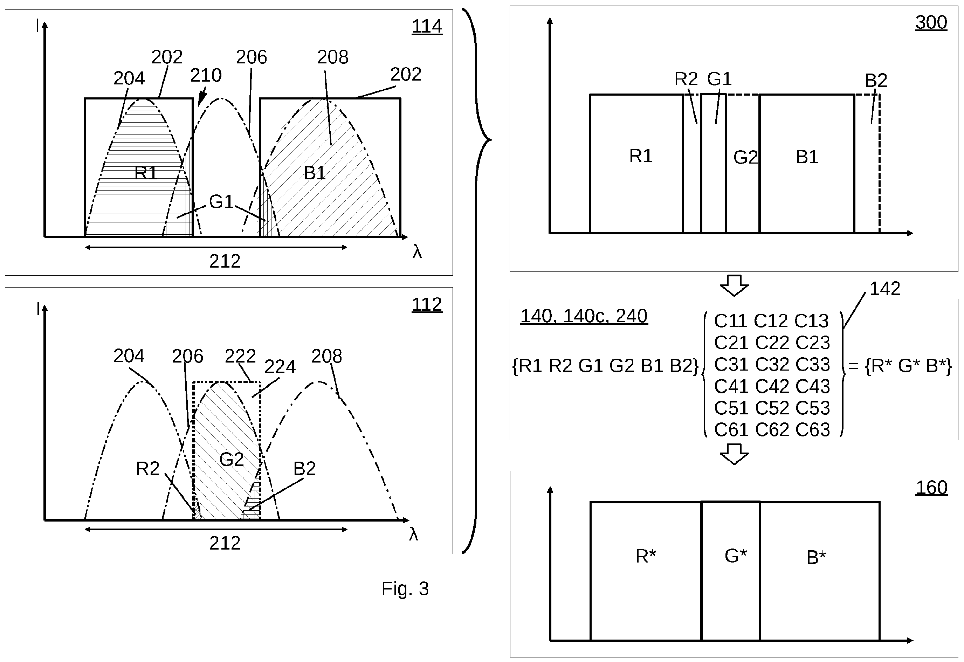

This is explained in the following with reference to Fig. 3. For simplicity's sake, Fig. 3 shows the spectra of Fig. 2 in an RGB color space and only a single passband 224 and stopband 210 are shown.

The digital white-light color image 114 recorded in the first imaged spectrum 202 is composed of signals R1, G1 and B1, each representing the intensity I in the respective color band. Due to the stop band 210, the signal G1 results from two separated wavelengths bands. The sensor recording G1 however cannot distinguish between these two wavelength bands.

The digital fluorescence-light color image 112 recorded in the second imaged spectrum 222 is composed of signals R2, G2, B2 in each color band.

As the first and second imaged spectrum 202, 222 are complementary to each other, each color band is split into two signals, respectively, each signal coming from a different camera 110, 111 and representing or being equivalent to a sub-band of the color band. The color band 204 is split into R1 and R2, respectively. The color band 206 is split into G1 and G2, respectively, and the color band 208 is split into B1 and B2 respectively. Preferably, there is no overlap between the various signals in a color band.

Thus, even if the first and second imaged spectrum 202, 222 are recorded in the same color space using the same type of color cameras, a multispectral image of the object 106 results, which is composed in this case of six color bands R1, R2, G1, G2, B1, B2. This is shown schematically at reference numeral 300. It is to be noted that this is independent of whether the fluorescence camera actually records fluorescence emission or reflectance.

In other words, at least one color band 204, 206, 208 of the color space at least of the digital output color image 160 is subdivided into two sub-bands R1, R2, G1, G2, B1, B2, wherein one of the two sub-bands is comprised in the digital fluorescence-light color image 112 and the other of the two sub-bands is comprised in the digital white-light color image 114. Preferably, the two sub-bands in a color band do not overlap. They are preferably complementary. Most preferably, they, together, at least almost complete the respective color band. A sub-band may comprise or consist of two separated spectral bands. Each sub-band may be considered as being itself a color band.

The color conversion function 140 reflects the subdivision of the color bands into the sub-bands that is determined by the stop-band 210 and the pass-band 224. As the widths and/or locations of the sub-bands determine how much light will be gathered by the respective camera 110, 111 in that sub-band, the color conversion function 140c needs to be adjusted for each different filter setting of the color separation assembly 176.

Thus, the digital white-light image 112 and the digital fluorescence-image 114 may be processed together as an image composed of the sum of color bands in the two images 112 and 114. This provides improved color resolution.

For example, the color conversion function 140c may be applied in the form of a linear transformation using the color conversion matrix 142 to generate the digital output color image 160.

If the digital output color image 160 is represented in an RGB color space, a 6x3 or 3x6 color conversion matrix 142 may be applied to the combined digital fluorescence-light and white light image 112, 114 or their constituent signals R2, G2, B2, R1, G1, B1, respectively to arrive at RGB signals R*, G*, B*. The matrix coefficients C11, C12, ..., C63 may be determined by experiment.

It is to be noted that the color conversion matrix 142 may in effect result from applying several matrices one after the other.

In Fig. 3, an input union or, synonymously, an input union set {R1, R2, G1, G2, B1, B2} is formed from the set of color space coordinates {R1, G1, B1} in the color bands R, G, B of a (first) pixel of the digital white-light color image 114 and the set of color space coordinates {R2, G2, B2} in the color bands R, G, B of a preferably corresponding (second) pixel of the digital fluorescence-light color image 112. The input union set corresponds to a pixel of a multispectral image. The color conversion matrix is then applied to the input union. For forming the input union, no additional memory is needed. The input union may be generated logically by e.g. combining pointers to the color space coordinates of the first and second pixel. Of course, the color space coordinates of these two pixels may also be copied to a memory which then physically stores the input union.

Next, operational mode B is explained.

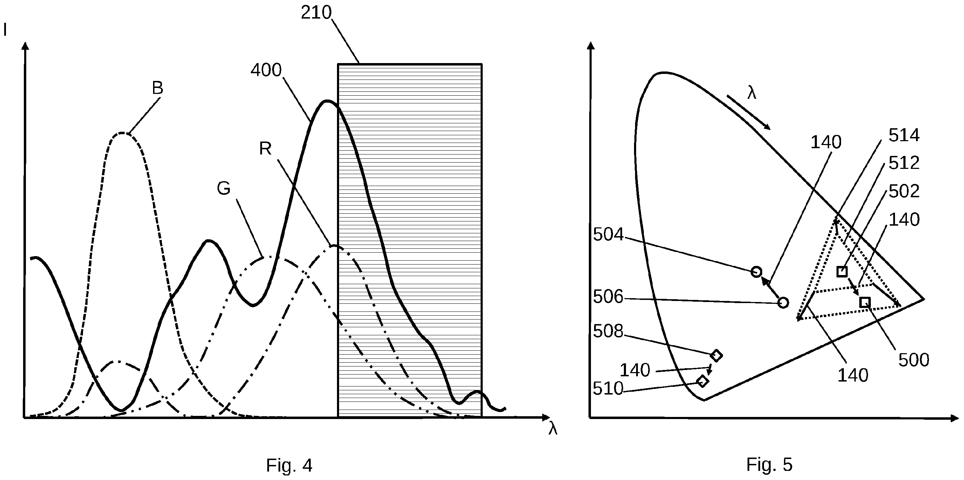

In Fig. 4, a schematic reflectance spectrum 400 of a biological tissue of the object 106 is indicated, wherein the term tissue comprises both fluid tissue and solid tissue. For example, the reflectance spectrum 400 may correspond to the reflectance spectrum of oxygenated blood.

The spectral sensitivities R, G, B of the R, G, B sensors of a sample RGB color camera are also shown in Fig. 4.

In human perception, tissue such as oxygenated blood is perceived as having a natural color, namely, red under white-light illumination, such as a CIE illuminant. Thus, the reflectance spectrum 400 is perceived as a single natural color although it is widely spread across the spectrum of visible light. In the first imaged spectrum 202, in which the digital white-light color image 114, is recorded, wavelengths λ in the stop band 210 are not recorded. Thus, the reflectance spectrum 400 as represented in the digital white-light color image 114 does not faithfully represent the natural color of the tissue.

The same applies to the second imaged spectrum 222 and the digital fluorescence-light color image 114, in which a reflectance spectrum 400 will also be distorted.

The same holds if the spectrum 400 is not a reflectance spectrum but a fluorescence emission spectrum 226 which is recorded in both the digital white-light color image 114 and the digital fluorescence-light color image 112 as shown in Fig. 2. The peak intensity and/or the larger part of the fluorescence-light energy of the fluorescence emission spectrum will be within the stop band 210 and thus be recorded by the fluorescence-light camera.

Thus, the case where both the fluorescence-light color camera 111 and the white-light color camera 110 are used to record solely fluorescence corresponds to the case where both cameras record white-light reflectance. In the former case, the spectral fluorescence information is mainly recorded by the fluorescence-light color camera 111 and supplemented by the spectral information recorded by the white-light color camera 110; in the latter case, the spectral white-light reflectance information is mainly recorded by the white-light color camera 110 and supplemented by the spectral information recorded by the fluorescence-light color camera 111.

Fig. 5 shows a schematic rendition of a CIE 1932 color space diagram, in which a color 500 is shown. The color 500 may for example represent the natural color which is perceived by a standard observer when looking at the tissue having the reflectance spectrum 400 (Fig. 4) illuminated by a CIE illuminant A, B or C or another standard illuminant.

As the wavelengths in the stopband 210 are not recorded, the color 500 is represented in the digital white-light color image 114 as a (recorded) color 502 in the digital white-light color image 114. In order to more faithfully represent color 500, the recorded color 502 is converted to color 500 by a color conversion function 140, which may be a first or a second color conversion function 140a, 140b. For example, the color conversion may be a shift or translation in LMS color space or tristimulus coordinates.

The color conversion function 140 may be determined by a color calibration using known colors, e.g. on color cards, known filter settings and known illumination conditions. The color 500 may be of a wavelength that is in the stopband 210 and thus not within the first imaged spectrum 202. Together with the shift from 502 to 500, the entire color space may be shifted.

It may be beneficial in some applications if the color 500, to which color 502 is converted by the color conversion function, is not a natural color, but a pseudocolor or a color which is close to but visibly different to the natural color. This may help to offset this color and the associated tissue type from other tissues or fluorescence colors that are close to color 500, and to highlight this type of tissue to the experienced eye. For example, the color 500 in the case of oxygenated blood may be a "hyperreal" color, e.g. of a brighter red than the natural color of oxygenated blood, or a red pseudocolor such as neon red. Of course, such a conversion can be done for any other color or tissue type as well.

Due to the stopband 210, the location of the natural white point 504 is shifted to recorded white point 506. Thus, another color conversion function 140, e.g. color conversion function 140c, may shift the recorded white point 506 to or closer to the natural white point 504. In this respect, a white point is treated like any color.

A shift of a single color may correspond to a shift of the entire color space, i.e. all colors are shifted by the same amount.

A shift of the entire color space by the same amount and the same direction may be used to white-balance or color-balance the digital white-light color image 112.

Alternatively, different colors or different sets of colors may be converted or shifted differently with respect to at least one of the amount and the direction of the shift. For example, the recorded color 508 of e.g. deoxygenated blood may be shifted further into the blue color range, into color 510. The shift of color 508 to 510 may differ from the shift of color 502 to color 500. Thus, instead of translating the entire color space, a color conversion function 140 may be configured to generate a color-depending conversion. For different (recorded) colors 502, 504, 508, different color conversion functions 140 may be applied.

Another example of a color conversion function 140, e.g. 140d, may expand a region 512 in the color space to a larger region 514. In region 514, the distance between colors is larger than in region 512, so that differences in colors become more visible. The application of such a color conversion function may be color-dependent. In this case, only the region 512 of predetermined colors is expanded. This color conversion function 140 may include a color shift, so that the region 514 is not only expanded but also moved to a different region of the color space.

Any of the above color conversion functions 140 may convert only a selected subset of the color appearance parameter of a color. The color appearance parameters are hue, colorfulness, saturation, lightness and brightness. For example, only the hue may be changed when converting color 502 to color 500, or, only saturation and lightness are changed by a color conversion function 140.

Any combination of the above color conversion functions 140a may be applied. It is to be understood, that although the above description uses the expression "image", the color conversion function 140 is applied to the image on the pixel level, i.e. to each pixel 150 of the digital white-light color image 112. The color of the pixel 150 may determine which color conversion function 140 or which combination of color conversion functions 140 is applied. This can done automatically by the processor 170.

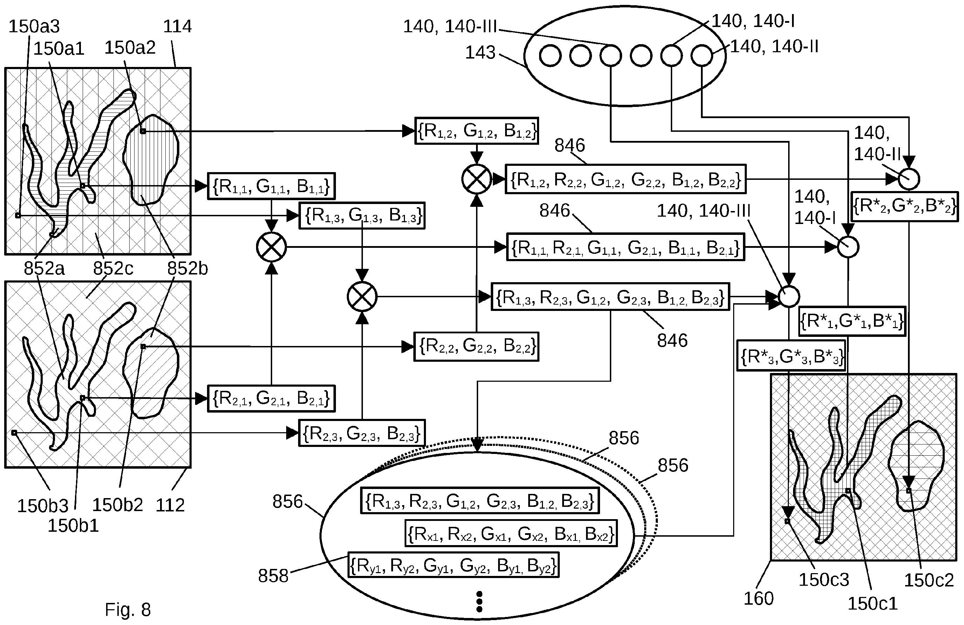

In Fig. 8, an example of a color-dependent color conversion is shown. The digital white-light color image 114 and the digital fluorescence-light color-image 112 may, in this example both be reflectance images of the object 106. However, this does not need to be the case, and the digital fluorescence image 112 may be instead be a fluorescence image or a reflectance image, in which e.g. only fluorescence of a fluorophore is contained.

In the images 112, 114, three different tissue types 852a, 852b, 852c are shown. Of course, more or less tissue types may be present in the images 112, 114. The various tissue types may not be clearly visible in one or both images 112, 114. For example, tissue 852a may be an arterial blood vessel comprising oxygenated blood, tissue 852b may be a venous blood vessel comprising de-oxygenated blood or, if fluorescence is recorded in the digital fluorescence-light image, a tumor and 852c may be background, e.g. in brain surgery, neural tissue.

A pixel 150a1 of the digital white-light color image 114 is located in the area of tissue 852a. The color space coordinates of pixel 150a1 are, if an RGB color space is assumed for explanatory purposes only, {R_{1,1}, G_{1,1}, B_{1,1}}. A pixel 150a2 of the digital white-light color image 114 is located in the area of tissue 852b. The color space coordinates of pixel 150a2 are {R_{1,2}, G_{1,2}, B_{1,2}}. A pixel 150a3 of the digital white-light color image 114 is located in the area of tissue 852c. The color space coordinates of pixel 150a3 are {R_{1,3}, G_{1,3}, B_{1,3}}. The color space coordinates are recorded in the first imaged spectrum 202.

In the digital fluorescence-light image 112, pixel 150b1 is located in tissue 852a and preferably is a corresponding pixel to pixel 150a1, pixel 150b2 is located in tissue 852b and preferably is a corresponding pixel to pixel 150a2, and pixel 150b3 is located in tissue 852c and preferably is a corresponding pixel to pixel 150a3. Corresponding pixels are preferably located at corresponding locations of the images 112, 114 or of a pattern that has been previously identified in the images 112, 114 using a pattern recognition algorithm.

The color space coordinates of pixel 150b1 are, if again an RGB color space is assumed, {R_{2,1}, G_{2,1}, B_{2,1}}. The color space coordinates of pixel 150b2 are {R_{2,2}, G_{2,2}, B_{2,2}}. The color space coordinates of pixel 150b3 are {R_{2,3}, G_{2,3}, B_{2,3}}. These color space coordinates were recorded in the second imaged spectrum 222 and therefore contain different spectral information than the color space coordinates of the corresponding pixels 150a-c in the digital white-light color image. If the color spaces of the images 112, 114 are different, they may be converted into a common color space.

As in Fig. 3, an input union (set) 846 of the color space coordinates may be formed at each of the pixels 150a1-150a3 and 150b1-150b3. Thus, the color space coordinates pixels 150a1 and 150b1, {R_{1,1}, G_{1,1}, B_{1,1}} and {R_{2,1}, G_{2,1}, B_{2,1}} form the input union {R_{1,1}, R_{2,1}, G_{1,1}, G_{2,1}, B_{1,1}, B_{2,1}}. The color space coordinates pixels 150a2 and 150b2, {R_{1,2}, G_{1,2}, B_{1,2}} and {R_{2,2}, G_{2,2}, B_{2,2}} form the input union {R_{1,2}, R_{2,2}, G_{1,2}, G_{2,2}, B_{1,2}, B_{2,2}}. The color space coordinates pixels 150a3 and 150b3, {R_{1,3}, G_{1,3}, B_{1,3}} and {R_{2,3}, G_{2,3}, B_{2,3}} form the input union {R_{1,3}, R_{2,3}, G_{1,3}, G_{2,3}, B_{1,3}, B_{2,3}}. Note that each union 846 essentially corresponds to the input union {R1, R2, G1, G2, B1, B2} of Fig. 2. Each different input union 846 corresponds to a different color.

A set 143 comprising at least two, i.e. a plurality of, color conversion functions 140 may be provided, e.g. stored in the image processor 170. The set 143 may, for example, comprise the different color conversion functions 140-1, 140-II, 140-III shown in Fig. 5. Instead of a set, a single color conversion function 140 may be provided. The color conversion functions 140-1, 140-II, 140-III may correspond to the color conversion functions 140a, 140b, 140c in one embodiment.

If only a single color conversion function 140 is provided, its application may depend on the input union 846 in Fig. 8, i.e. the color space coordinates contained therein. If, for example, the input union belongs to a predetermined set 856 of target unions 858, which is or has been assigned to a specific color conversion function 140, this specific color conversion function 140 is applied to the input union. Otherwise, the color conversion function 140 is not applied. In this case, the color conversion function 140 may only be applied if the input union 846 represents a color that is typical for tissue 852a but not of tissue 852b or 852c, because the color conversion function 140 is or has been assigned to the colors that are representative of tissue 852a.

If two or more color conversion functions 140 are provided, e.g. color conversion functions 140-1, 140-II, 140-III, it may depend on the input union 846, i.e. the set of color space coordinates of the input union, which of the color conversion functions 140-1, 140-II, 140-III or whether any color conversion function at all is applied. Each set 856 is assigned to a different color conversion function.

For example, if the input union 846, or, more exactly, a target union 858 which corresponds to the input union 846, is contained in a predetermined set 856 of target unions 858, the color conversion function 140-1 assigned to this particular set 856 may be applied. If the input union 846 is contained in a second predetermined set of target unions 858 preferably not overlapping the first set, the color conversion function 140-11 assigned to this set may applied. If the input union 846 is contained in a third predetermined set 856 of target unions, a third color conversion function 140-III or no color conversion function may be applied. Or, if the input union 846 is not contained in any set 856, no, or a specific color conversion function may be applied. Thus, the tissue 852a may undergo a different color conversion than tissue 852b and/or tissue 852c, as explained in Fig. 5 with reference to the colors 500, 508, 504, each of which may be represented by a different input union.

Each set 856 represents a different group of colors. Each set 856 may in particular a specific type of tissue which exhibits colors contained in this set. For example, one set 856 may comprise a range of brighter reds for representing oxygenated blood, another set 856 may comprise a range of bluish reds to represent de-oxygenated blood, another set 856 may comprise a range of greyish pinks to represent live grey brain matter, another set 856 may comprise a range of whitish pink colors to represent live white brain matter, and so on. Any number and combination of sets may be used. A target union 858 should, however, be contained only in one set 856 to have a one-to-one assignment of color conversion functions and input unions 846.

The various sets 856 and their target unions 858 may represent different types of fluorescence if the digital white-light color image 114 and the digital fluorescence-light color image 112 record only fluorescence. For example, one set 856 may contain target unions which map the fluorescence of a fluorophore, such as 5-ALA/pPiX to an output color. Another set 856 may contain target unions 858 of which are representative for auto-fluorescence of biological tissue and map their color as represented in the input union to either a reflectance color of the biological tissue or a natural-looking auto-fluorescence color or a pseudo color. Again another set 856 may contain target unions 858 which represent the reflectance colors of the object under illumination of the fluorescence emission spectrum and map them to more natural colors.

The target unions 858 may be determined empirically in a calibration process.

The at least one set 856 of target unions 856 may be stored in or comprised by the image processor 170 e.g. as a look-up table.

The color conversion function 140-1 that is assigned and applied to the input union {R_{1,1}, R_{2,1}, G_{1,1}, G_{2,1}, B_{1,1}, B_{2,1}} results in a conversion of the input union to the color space coordinates {R*₁, G*₁, B*₁} which are assigned to pixel 150c1 in the digital output color image 150. Output pixel 150c1 is preferably a corresponding pixel to at least one of the pixels 150a1 and 150b1. The color conversion function 140-11 that is assigned and applied to the input union {R_{1,2}, R_{2,2}, G_{1,2}, G_{2,2}, B_{1,2}, B_{2,2}} results in a conversion of the input union to the color space coordinates {R*₂, G*₂, B*₂} which are assigned to pixel 150c2 in the digital output color image 150. Output pixel 150c2 is preferably a corresponding pixel to at least one of the pixels 150a2 and 150b2. The color conversion function 140-III that is assigned and applied to the input union {R_{1,3}, R_{2,3}, G_{1,3}, G_{2,3}, B_{1,3}, B_{2,3}} results in a conversion of the input union to the color space coordinates {R*₃, G*₃, B*₃} which are assigned to pixel 150c3 in the digital output color image 150. Output pixel 150c1 is a corresponding pixel to at least one of the pixels 150a3 and 150b3. If no color conversion function is assigned to an input union and consequently no color conversion applied to this input union, then the color space coordinates in the digital output color image 160 may be computed by adding the color space coordinates in the union which are in the same color band, as was explained with reference to Fig. 2.

As the input union comprises much more color bands than each of the images 112, 114, the accuracy of the union-dependent color conversion matches the accuracy of tissue-type detection. In fact, the tissue-type dependent color conversion may be integrated into the color conversion process by accurately calibrating the different predetermined sets 856 of target unions 858 which determine which color conversion function is used.

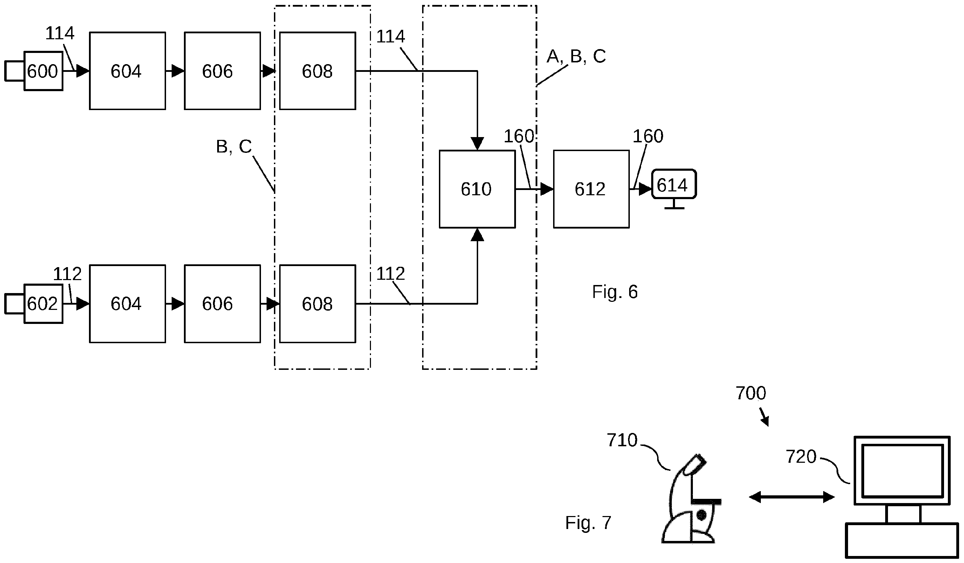

Fig. 6 shows a schematic representation of the imaging method that may be implemented as a computer-implemented method running e.g. on the image processor 170.

At optional step 600, a digital white-light color image 114 is recorded, using e.g. the white-light color camera 110. At optional step 602, a digital fluorescence-light color image 112 is recorded, using e.g. the fluorescence-light camera 111. Steps 600 and 602 are optional because the images 112, 114 may also be retrieved from memory. As explained above, the cameras 110, 111 should be synchronized with respect to exposure time and locked to one another with respect to gain, i.e. maintain the same gain ratio. Gamma may be set to a fixed value and all automatic color adjustments are preferably turned off.

At optional step 604, the respective image 112, 114 may be demosaiced.

At optional step 606, one or both images 112 or 114 may be matched and registered so that identical image features are represented in each image 112, 114 geometrically identically with respect to location, size and orientation. After registration, each pixel in the digital white-light color image 114 has a corresponding pixel in the digital fluorescence-light color image 112. Preferably, corresponding pixels are located at the same position in each of the images 112, 114.

In one operational mode, e.g. operational mode B, but optionally also in operational mode C, a color conversion is performed in optional color conversion step 608. Step 608 is e.g. omitted in operational mode A. In step 608 a color conversion function 140 or a combination of color conversion functions 140 as described above is applied to at least one of the digital white-light color image 112 and the digital fluorescence-light image 114.

At image-combining step 610, the digital white-light color image 114 and the digital fluorescence-light color image 112 are combined to generate the digital output color image 160. This may take place in all operational modes A, B, C. As described above, the images 112, 114 may for example simply be added for combination, as shown in Fig. 2. Such an addition may be performed by adding the color space coordinates of the two images that are located in the same color band. If a pixel in the digital white-light color image 114 has color space coordinates {R1, G1. B1} in a color space and the corresponding pixel in the digital fluorescence-light color image 112 has color space coordinates {R2, G2, B2}, then the color space coordinates {R*, G*, B*} of the corresponding pixel in the digital output color image may be calculated as R*=R1+R2, G*=G1+G2, B*=B1+B2.

In operational mode C, a color conversion function 140 may be applied to both the digital white-light color image 114 and the digital fluorescence-light image 112 in step 610 to generate the digital output color 160, as shown in Fig. 3. In step 610 a color conversion function may be selected from a predetermined set of color functions 140 depending on the input union and applied to the input union as was explained with reference to Fig. 8.

In step 612, post-processing may be carried out. For example, the digital output color image 160 may be homogenized, its contrast may be enhanced and/or color-space conversions such as from RGB to sRGB, and/or gamma corrections may be performed. It is important to note that the color conversion in step 608 and/or 610 is not a gamma correction.

At step 612, the digital output image 160 is displayed.

Some embodiments relate to a microscope, in particular a fluorescence microscope comprising a system as described in connection with one or more of the Figs. 1 to 6. Alternatively, a microscope may be part of or connected to a system as described in connection with one or more of the Figs. 1 to 6.

The medical fluorescence observation device 100 may be configured to select which of a set 143 of different color conversion functions 140, e.g. 140-1, 140-II, 140-III is applied to at least one of the digital fluorescence-light color image 112 and the digital white-light color image 114. More specifically, the input selector may be configured to perform this selection.

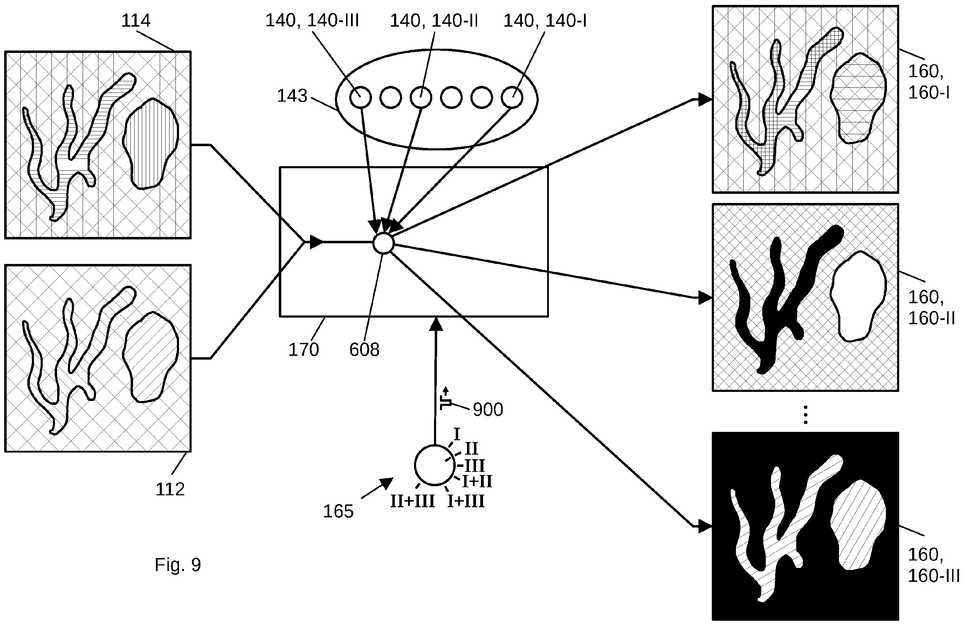

Further, the medical fluorescence observation device 100 may be configured to select which of the digital output color images 160 is displayed in a display 132, 182, for example the eyepiece, VR goggles and/or an external monitor. Again, the selector device may be configured to select which of the digital output color images 160 is displayed on the display 132, 182. This is now explained with reference to Fig. 9, where the image processor 170 is shown to be configured to apply different color conversion functions 140-1, 140-II, 140-III of one or more sets 143 of color conversion functions to the to the digital fluorescence-light color image 112 and/or the digital white-light color image 114 as explained above. Any number of different color conversion functions 140 and/or of sets 143 may be used.

Each color conversion function 140-1, 140-II, 140-III, if applied to the digital fluorescence-light color image 112 and/or the digital white-light color image 114 e.g. in the color conversion step 608, results in the generation of a different digital color output image 160-1, 160-II, 160-III. For example, if the color conversion function 140-1 is applied to the to the digital fluorescence-light color image 112 and/or the digital white-light color image 114, the digital output color image 160-I is generated; application of the color conversion function 140-11 results in the generation of the digital output color image 160-11 and so on.

The selector device 165 is shown as being connected to the image processor 170. A display selection signal 900 may be sent from the selector device 165 to the image processor 170 by any kind of wireless and/or wired transmission path.

The selector device 165 is a piece of hardware, such as a knob or switch. However, the selector device 165 may also be a software device, such as a graphical interactive element which may be operated by the user similar to a hardware switch, and reside at least partly in the image processor 170. The selector device may also be a combination of hardware and software, the software part may, in this case, also reside at least partly in the image processor 170.

The selector device 165 may, as was described above, be used to select different modes of operation of the medical fluorescence observation device 100. The display selection signal 900 may be representative of at least some of these different modes of operation.

In Fig. 9, each different mode of operation involves the application of a different color conversion function 140 or different set of color conversion functions 140. For example, the selector device 165 may be used to select six different modes of operations such as mode I, mode II, mode III, mode I+II, mode I+III and mode II+III. In mode I, color conversion function 140-1 is applied, whereas in mode II, color conversion function 140-11 is applied and in mode III, color conversion function 140-III is applied. In mode I+II, color conversion functions I and II are applied, in mode I+III, color conversion functions 140-1 and 140-III are applied an in mode II+III color conversion functions 140-11 and 140-III are applied. Of course, there may also be a mode I+II+III, where color conversion functions 140-1, 140-11 and 140-III are applied.

In mode I+II and the other modes, where two or more different color conversion functions are selected for operation on the digital white-light color image 114 and/or the digital fluorescence-color image 112, each color conversion function may be successively applied on top of one another. For example, in mode I+II, first the color conversion function 140-1 is applied to the the digital white-light color image 114 and/or the digital fluorescence-color image 112, then the color conversion function 140-11 is applied to the result.

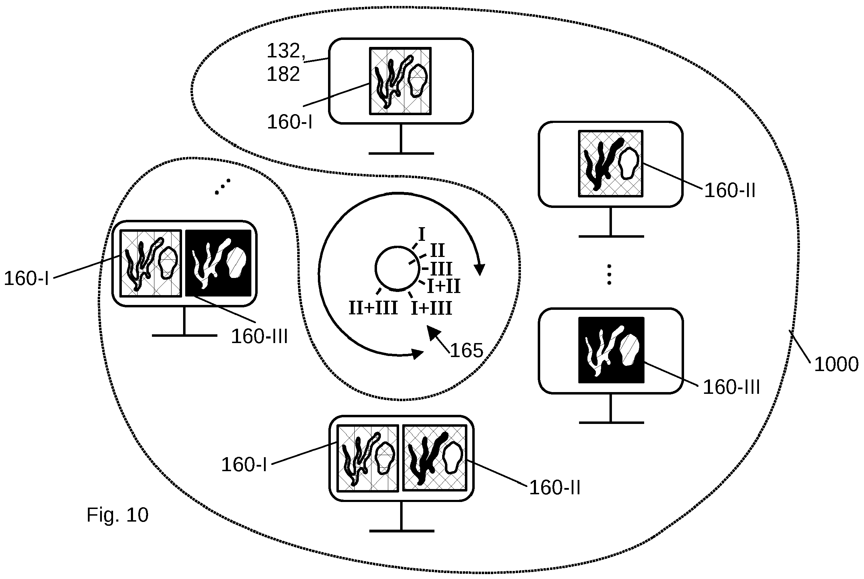

In another variant, shown in Fig. 10, the selector device 165 may be used to select which of the different digital output color images 160-1, 160-11 and 160-III is displayed on display 132, 182. If I is selected on the selector device, digital output color image 160-1 is displayed, if II is selected on the selector device, digital output color image 160-11 is displayed and so on. Further, if I+II is selected, digital output color images 160-1 and 160-11 are displayed simultaneously on separate parts of the display 132, 182, e.g. side by side. If II, III is selected, digital output color images 160-II and 160-III are displayed simultaneously on separate parts of the display 132, 182. This may be continued for any number and combination of color conversion functions 140 and digital color output images 160.

Thus from the set 143 of color conversions, an output set 1000 of different digital output color images 160 may be generated. The image processor 170 is configured to select at least one digital output color image from the output set 1000 for display depending on the display selection signal 900. The selector device 165 may be configured to generate a plurality of different display selection signals 900, where each selector signal may be uniquely assigned to a digital output color image 160 or combination of digital output color images 160 of the output set 1000. The image processor is preferably configured to display the digital output color image 160 assigned to the display selection signal 900, which has been received last.

If more than one digital output color image 160 is selected to be displayed, the displayed digital output color images 160 are preferably synchronized, i.e. generated from the same digital white-light color image 114 and the digital fluorescence-color image 112.

In one embodiment, the digital output color image 160-1 may be generated only from the digital fluorescence-light color image 112 as described above, which may in particular represent the fluorescence emission of at least one fluorophore 116. In this case, a color conversion function 140-1 may be applied only to the fluorescence-light color image 112, which color conversion function enhances contrast of the fluorescence.

In another embodiment, the digital output color image 160-11 may be generated from a combination of the digital white-light color image 114 and the digital fluorescence-light color image 112 as described above where the digital white-light color image 114 represents a reflectance image of the object 106 under illumination of the fluorescence excitation spectrum and the digital fluorescence-light color image 114 represents the fluorescence emission of the at least one fluorophore. In this case, a color conversion function 140-11 may be applied only to the white-light color image 114. Such a color conversion function may be configured to shift the colors in the digital white-light color image to a more natural color.

In yet another embodiment, the digital output color image 160-III may be generated from a combination of the digital white-light color image 114 and the digital fluorescence-light color image 112. Here, the digital white-light color image 114 may represent a reflectance image of the object 106 under white-light illumination and the digital fluorescence-light color image 114 may represent the fluorescence emission of the at least one fluorophore. As described above, separate color conversion functions 140 may be applied to the digital fluorescence-color image 112 and the digital white-light color image 114, or a color conversion function may be applied to only one of the digital fluorescence-light color image 112 and the digital white-light color image 114, or to the combination of the the digital fluorescence-light color image 112 and the digital white-light color image 114.

In the combined view, any two of these three images may be combined depending on the selection by the user.

Fig. 7 shows a schematic illustration of a system 700 configured to perform a method described herein. The system 700 comprises a microscope 710 and a computer system 720. The microscope 710 is configured to take images and is connected to the computer system 720. The computer system 720 is configured to execute at least a part of a method described herein. The computer system 720 may be configured to execute a machine learning algorithm. The computer system 720 and microscope 710 may be separate entities but can also be integrated together in one common housing. The computer system 720 may be part of a central processing system of the microscope 710 and/or the computer system 720 may be part of a subcomponent of the microscope 710, such as a sensor, an actor, a camera or an illumination unit, etc. of the microscope 710.

The computer system 720 may be a local computer device (e.g. personal computer, laptop, tablet computer or mobile phone) with one or more processors and one or more storage devices or may be a distributed computer system (e.g. a cloud computing system with one or more processors and one or more storage devices distributed at various locations, for example, at a local client and/or one or more remote server farms and/or data centers). The computer system 720 may comprise any circuit or combination of circuits. In one embodiment, the computer system 720 may include one or more processors which can be of any type. As used herein, processor may mean any type of computational circuit, such as but not limited to a microprocessor, a microcontroller, a complex instruction set computing (CISC) microprocessor, a reduced instruction set computing (RISC) microprocessor, a very long instruction word (VLIW) microprocessor, a graphics processor, a digital signal processor (DSP), multiple core processor, a field programmable gate array (FPGA), for example, of a microscope or a microscope component (e.g. camera) or any other type of processor or processing circuit. Other types of circuits that may be included in the computer system 720 may be a custom circuit, an application-specific integrated circuit (ASIC), or the like, such as, for example, one or more circuits (such as a communication circuit) for use in wireless devices like mobile telephones, tablet computers, laptop computers, two-way radios, and similar electronic systems. The computer system 720 may include one or more storage devices, which may include one or more memory elements suitable to the particular application, such as a main memory in the form of random access memory (RAM), one or more hard drives, and/or one or more drives that handle removable media such as compact disks (CD), flash memory cards, digital video disk (DVD), and the like. The computer system 720 may also include a display device, one or more speakers, and a keyboard and/or controller, which can include a mouse, trackball, touch screen, voice-recognition device, or any other device that permits a system user to input information into and receive information from the computer system 720.

Some or all of the method steps may be executed by (or using) a hardware apparatus, like for example, a processor, a microprocessor, a programmable computer or an electronic circuit. In some embodiments, some one or more of the most important method steps may be executed by such an apparatus.

Depending on certain implementation requirements, embodiments of the invention can be implemented in hardware or in software. The implementation can be performed using a non-transitory storage medium such as a digital storage medium, for example a floppy disc, a DVD, a Blu-Ray, a CD, a ROM, a PROM, and EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments according to the invention comprise a data carrier having electronically readable control signals, which are capable of cooperating with a programmable computer system, such that one of the methods described herein is performed.

Generally, embodiments of the present invention can be implemented as a computer program product with a program code, the program code being operative for performing one of the methods when the computer program product runs on a computer. The program code may, for example, be stored on a machine readable carrier.

Other embodiments comprise the computer program for performing one of the methods described herein, stored on a machine readable carrier.

In other words, an embodiment of the present invention is, therefore, a computer program having a program code for performing one of the methods described herein, when the computer program runs on a computer.

A further embodiment of the present invention is, therefore, a storage medium (or a data carrier, or a computer-readable medium) comprising, stored thereon, the computer program for performing one of the methods described herein when it is performed by a processor. The data carrier, the digital storage medium or the recorded medium are typically tangible and/or non-transitionary. A further embodiment of the present invention is an apparatus as described herein comprising a processor and the storage medium.

A further embodiment of the invention is, therefore, a data stream or a sequence of signals representing the computer program for performing one of the methods described herein. The data stream or the sequence of signals may, for example, be configured to be transferred via a data communication connection, for example, via the internet.

A further embodiment comprises a processing means, for example, a computer or a programmable logic device, configured to, or adapted to, perform one of the methods described herein.

A further embodiment comprises a computer having installed thereon the computer program for performing one of the methods described herein.

A further embodiment according to the invention comprises an apparatus or a system configured to transfer (for example, electronically or optically) a computer program for performing one of the methods described herein to a receiver. The receiver may, for example, be a computer, a mobile device, a memory device or the like. The apparatus or system may, for example, comprise a file server for transferring the computer program to the receiver.

In some embodiments, a programmable logic device (for example, a field programmable gate array) may be used to perform some or all of the functionalities of the methods described herein. In some embodiments, a field programmable gate array may cooperate with a microprocessor in order to perform one of the methods described herein. Generally, the methods are preferably performed by any hardware apparatus.

### REFERENCE NUMERALS

- 100: medical fluorescence observation device
- 101L: stereoscopic subassembly for the left channel
- 101R: stereoscopic subassembly for the right channel
- 102: digital imaging system
- 104: eyepiece
- 106: object to be investigated
- 107: biological tissue
- 110: white-light color camera
- 111: fluorescence-light color camera
- 112: digital fluorescence-light color image
- 114: digital white-light color image
- 116: fluorophore
- 118: second fluorophore
- 132: integral/internal display
- 134: direct optical path
- 136: beam splitter
- 140: color conversion function
- 140a-140d: different color conversion functions
- 140-1, 140-II, 140-III: different color conversion functions
- 142: color conversion matrix
- 143: set of color conversion functions
- 143a-143c: different sets of color conversion functions
- 150: pixel
- 150a1-150a3: pixel in digital white-light color image
- 150b1-150b3: pixel in digital fluorescence-light color image
- 150c1-150c3: pixel in digital output color image
- 160: digital output color image
- 165: selector device
- 169: filter setting selector
- 170: image processor
- 172: output interface
- 174: objective
- 176: color-separation assembly
- 178: illumination assembly
- 179: illumination filter
- 180: illumination beam splitter
- 182: display
- 184: field of view
- 186: computer
- 188: white-light filter
- 190: fluorescence-light filter
- 192: dichroic beam splitter
- 194: memory
- 196: data transmission line
- 200: spectrum
- 202: first imaged spectrum
- 204, 206, 208: primary color or color band
- 210: stopband
- 212: visible spectrum or light range
- 220: spectrum
- 222: second imaged spectrum
- 224: passband
- 226: fluorescence-emission spectrum of a fluorophore
- 228: fluorescence emission spectrum of another fluorophore
- 230: part of fluorescence emission spectrum in color band 204
- 232: part of fluorescence emission spectrum in color band 206
- 236: part of fluorescence emission spectrum in color band 208
- 240: combining images
- 250: spectrum
- 260: part of fluorescence emission spectrum in first imaged spectrum
- 262: part of fluorescence emission spectrum in first imaged spectrum
- 300: multispectral image from digital white-light and fluorescence-light color image
- 400: reflectance spectrum
- 500: converted color
- 502: recorded color
- 504: natural white point
- 506: recorded white point
- 508: recorded color
- 510: converted color
- 512: recorded color region
- 514: target color region
- 600: recording a digital white-light color image
- 602: recording a digital fluorescence-light color image
- 604: demosaicing
- 606: registering
- 608: color conversion step
- 610: image combining step
- 612: post-processing
- 614: displaying
- 700: system
- 710: microscope
- 720: computer system
- 846: input union
- 852a-852b: tissue type
- 856: set of different target unions
- 858: target union
- 900: display selection signal
- 1000: output set of digital output color images
- λ: wavelength
- I: intensity
- A: first operational mode of processor
- B: second operational mode of processor
- C11, C12, ...,C63: coefficients of the color conversion matrix
- RGB: example of a color space
- R, G, B, R1, G1, B1, R2, G2, B2, R*, G*, B*: (intensity) signals in the respective color bands, or sub-bands of a color space

## Claims

1. Image processor (170) for a medical fluorescence observation device (100), such as a fluorescence microscope or a fluorescence endoscope, the image processor (170) being configured to:
- retrieve a digital white-light color image (114) of an object (106) recorded in a first imaged spectrum (202);
- retrieve a digital fluorescence-light color image (112) of the object (106) recorded in a second imaged spectrum (222), the second imaged spectrum (222) overlapping a fluorescence emission spectrum (226) of at least one fluorophore (116) and being different from the first imaged spectrum (202), the first and the second imaged spectrum (202, 222) both overlapping the visible spectrum (212); and
- output at least one digital output color image (160, 160-I, 160-II, 160-III) of a set (1000) of digital output color images (160) containing:
- a digital output color image (160, 160-1) generated by the image processor (170) from only the digital fluorescence-light color image (112);
- a digital output color image (160, 160-II) generated by the image processor (170) from only the digital white-light color image (114); and
- a digital output color image (160, 160-III) generated by the image processor (170) from a combination of the digital fluorescence-light color image (114) and the digital white-light color image (112);
wherein the image processor (170) is further configured to
- receive a display selection signal (900) from a selector device (165); and
- select the at least one digital output color image (160, 160-1, 160-II, 160-III) from the set for output depending on the display selection signal (900).

2. Image processor (170) according to claim 1, wherein the image processor (170)
- comprises a set (143) of color conversion functions (140, 140-I, 140-II, 140-III), each color conversion function being configured to map a color of an input pixel to a different color of an output pixel; and
- is configured to
∘ compute the digital output color image (160, 160-1) generated from only the digital fluorescence-light color image (112) by applying at least one color conversion function (140-1) of the set (143) of color conversion functions to the fluorescence-light color image (112);
∘ compute the digital output color image (160, 160-1) generated from only the digital white-light color image (114) by applying at least one color conversion function (140-II) of the set (143) of color conversion functions to the white-light color image (114);
∘ compute the digital output color image (160, 160-1) generated from the combination of the digital fluorescence-light color image (114) and the digital white-light color image (112) by applying at least one color conversion function (140-I. 140, II) of the set (143) of color conversion functions
to the white-light color image (114),
to the fluorescence-light color image (112) and/or
to the combination of the digital fluorescence-light color image (114) and the digital white-light color image (112).

3. Image processor (170) according to claim 1 or 2, wherein the image processor (170) is configured to output simultaneously at least two digital output color images of the set (1000).

4. Image processor (170) according to any one of claims 1 to 3, wherein the digital white-light color image (114) is representative of a reflectance image of an object (106) under illumination with a fluorescence excitation spectrum of at least one fluorophore (116), and/or the digital fluorescence-light color image (112) is representative of the fluorescence emission of the at least one fluorophore.

5. Image processor (170) according to any one of claims 1 to 3, wherein the digital white-light color image (114) is representative of a reflectance image of an object (106) under white-light illumination, and/or the digital fluorescence-light color image (112) is representative of the fluorescence emission of the at least one fluorophore.

6. Image processor (170) according to any one of claims 1 to 3, wherein the digital white-light color image (114) and the digital fluorescence-light color image (112) are representative of a reflectance image of an object (106) under white-light illumination.

7. Image processor (170) according to any one of claims 1 to 6, wherein the first imaged spectrum (202) and the second imaged spectrum (222) are complementary to one another.

8. Image processor (170) according to any one of claims 1 to 7, wherein the color conversion function (14) is a 3x3, a 6x3 or a 3x6 matrix.

9. Medical fluorescence observation device (100), such as a fluorescence microscope or a fluorescence endoscope, the medical fluorescence observation device (100) comprising:
an image processor (170) according to any one of claims 1 to 8;
a fluorescence-light color camera (111) configured to record the digital fluorescence-light color image (112); and
a white-light color camera (110) configured to record the digital white-light color image (114).

10. Medical fluorescence observation device (100) according to claim 9, wherein the medical fluorescence observation device (100) comprises a selector device (165) configured to be operated by a user of the medical fluorescence observation device (100) and to generate the display selection signal (900) depending on the operation of the user.

11. Medical fluorescence observation device (100) according to claim 9 or 10, wherein the medical fluorescence observation device (100) comprises at least one display (132, 182), which is connected to the at least one image processor (170), the display being configured to receive and display the at least one digital output color image (160) output by the image processor (170).

12. Medical fluorescence observation device (100) according to any one of claims 9 to 11, wherein the medical fluorescence observation device (100) is a surgical fluorescence microscope.

13. Computer-implemented image processing method for a fluorescence observation device (100), such as a fluorescence microscope or a fluorescence endoscope, the computer-implemented image processing method comprising the steps of:
- retrieving a digital white-light color image (114) of an object (106) recorded in a first imaged spectrum (202);
- retrieving a digital fluorescence-light color image (112) of the object (106) recorded in a second imaged spectrum (222), the second imaged spectrum (222) overlapping a fluorescence emission spectrum (226) of at least one fluorophore (116) and being different from the first imaged spectrum (202), the first and the second imaged spectrum (202, 222) both overlapping the visible spectrum (212); and
- outputting at least one digital output color image (160, 160-1, 160-II, 160-III) of the set (1000) of digital output color images (160) containing:
- a digital output color image (160, 160-1) generated by the image processor (170) from only the digital fluorescence-light color image (112);
- a digital output color image (160, 160-II) generated by the image processor (170) from only the digital white-light color image (114); and
- a digital output color image (160, 160-III) generated by the image processor (170) from a combination of the digital fluorescence-light color image (114) and the digital white-light color image (112);
wherein the computer-implemented image processing method further comprises the steps of:
- receiving a display selection signal (900) from a selector device (165); and
- selecting the at least one digital output color image (160, 160-1, 160-II, 160-III) from the set for output depending on the display selection signal (900).

14. A computer program product or a computer-readable medium comprising instructions which, when the program is executed by a computer, cause the computer to carry out the method of claim 13.

15. Method for operating a medical fluorescence observation device (100), such as a fluorescence microscope or a fluorescence endoscope, the method comprising the steps of:
- recording the digital fluorescence-light color image (112) in the second imaged spectrum (222) using a fluorescence-light color camera (111); and
- recording the digital white-light color image (114) in the first imaged spectrum (202) using a white-light color camera (110); and
- carrying out the computer-implemented image processing method of claim 13.
